# EUROPÄISCHE PATENTANMELDUNG

(11) **EP 4 083 183 A1**
(43) Veröffentlichungstag der Anmeldung: **02.11.2022**
(21) Anmeldenummer: 21170740.1
(22) Anmeldetag: 27.04.2021
(51) Int. Cl.: C12M 1/12, C12M 1/00, C12M 1/34, C12M 1/36

(54) **INKUBATOR FÜR ZELLKULTUREN**

(71) Anmelder: Eppendorf SE, 22339 Hamburg (DE)
(72) Erfinder: PAULSEN, Benjamin, 23569 Lübeck (DE); JOLIE, Christoph, 22765 Hamburg (DE); BECHMANN, Gregor, 22159 Hamburg (DE)
(74) Vertreter: Kirchner, Christian

(57) **Zusammenfassung**

Die Erfindung bezieht sich auf einen Inkubator mit Sensoreinrichtung zur Erfassung einer VOC-Kontamination der Gasatmosphäre des Innenraums der Inkubatorkammer.

## Beschreibung

Die Erfindung betrifft einen Inkubator für das Wachstum von biologischen Zellen. Die Erfindung betrifft zudem ein System und ein Verfahren zur Messung einer Inkubatoratmosphäre.

Mit solchen Inkubatoren werden in biologischen und medizinischen Laboratorien Zellen in Zellkultur unter kontrollierten Umgebungsbedingungen gehalten, und so das Wachstum lebender Zellen in vitro ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen CO2-Inkubatoren. Die Atmosphäre wird durch Luft mit einem bestimmten CO2- und O2-Gehalt und einer bestimmten Luftfeuchtigkeit gebildet, eine geeignete Temperatur ist oftmals 37 °C.

Ein Inkubator stellt nicht nur für Zellkulturen eine ideale Wachstumsumgebung dar, sondern auch für biologische Kontaminanten. Die Folgen biologischer Kontaminationen von Zellkulturen verursachen weitreichende Probleme in der biologischen Forschung, Impfstoffproduktion, personalisierten Medizin und regenerativen Medizinapplikationen. Folgen von biologischer Kontamination sind somit Verlust von Zeit und Geld, inakkurate bzw. fehlerhafte experimentelle Ergebnisse. Es gibt auch Fälle, beispielsweise in der Forensik oder der Reproduktionsmedizin, in denen der Wert einer einzelnen Probe, insbesondere in einem Zellkulturgefäß befindlichen Zelle/n, viel höher einzuschätzen ist als, beispielsweise, der Wert des gesamten Inkubators, so dass ein kontaminationsbedingter Verlust der Probe unbedingt zu vermeiden ist.

Kontamination der Inkubatorkammer kann aber auch nicht-biologische Ursachen haben, und kann insbesondere eine aus der Umgebung des Inkubators in die Inkubatorkammer hineingetragene Kontamination sein. Möglich ist eine nicht-biologische Kontamination beispielsweise als eine Folge der Herstellung des Inkubators, wenn beispielsweise organische Substanzen aus Kunststoffen, insbesondere aus Isolier- und/oder Dichtungsmaterial ausdampfen bzw. freigesetzt werden und in die Inkubatorkammer eindringen.

Der Funktionsumfang von Inkubatoren des Stands der Technik umfasst oftmals auch das Erhitzen der Inkubatorkammer z.B. auf 180 °C zum Zwecke einer Hochtemperatursterilisation. Bei der Hochtemperatursterilisation darf die Inkubatorkammer keine hitzeempfindlichen Gegenstände beinhalten. Es muss deshalb die Inkubatorkammer vor einer Hochtemperatursterilisation ausgeräumt werden, und die empfindlichen Proben müssen dabei in mindestens einen zweiten Inkubator umgelagert werden. Durch diese zusätzliche Manipulation der Proben besteht ein erhöhtes Risiko für einen Probenverlust. Zudem entsteht das Risiko, eine im Inkubator eventuell entstandene Kontamination durch die umgeräumten Probenbehälter in den mindestens einen zweiten Inkubator einzuschleppen, und gegebenenfalls noch weiter zu verbreiten. Eine umsichtig durchgeführte Hochtemperatursterilisation in Kombination mit der Probenumlagerung bedeuten in jedem Fall einen erheblichen Aufwand. Aufgrund der Bedeutung der Sterilität der Inkubatorkammer gehört es dennoch zur Standardempfehlung der Hersteller, die Hochtemperatursterilisation in regelmäßigen Abständen, z.B. zweimal pro Monat, durchzuführen. Auf diese Weise wird statistisch gesehen das Risiko für einen kontaminationsbedingten Probenverlust reduziert. Wünschenswert wäre es, den Aufwand der Anwender für ein möglichst steriles Betreiben der Inkubatorkammer zu reduzieren.

Der vorliegenden Erfindung liegt daher die Aufgabe zugrunde, einen Inkubator bereitzustellen, dessen Inkubatorkammer vom Anwender mit möglichst geringem Aufwand zuverlässig kontaminationsarm betrieben werden kann.

Die Erfindung löst diese Aufgabe durch den Inkubator nach Anspruch 1, das Verfahren nach Anspruch 16 und eine nachrüstbare Sensoreinrichtung nach Anspruch 17. Bevorzugte Ausgestaltungen sind insbesondere Gegenstände der Unteransprüche.
Der erfindungsgemäße Inkubator zur Inkubation lebender Zellkulturen, weist auf: eine Inkubatorkammer zur Aufnahme von Objekten, insbesondere Zellkulturbehältern, in einem verschließbaren Innenraum der Inkubatorkammer, die mit einer kontrollierten Gasatmosphäre betreibbar ist, eine Sensoreinrichtung zur Detektion einer Anreicherung, insbesondere Kontamination, von flüchtigen organischen Verbindungen (VOCs) in der Gasatmosphäre des Innenraums, die mindestens einen VOC-Sensor zur Detektion der VOCs aufweist, der einen Messbereich aufweist, welcher in Strömungsverbindung mit dem Atmosphärengas des Innenraums stehend angeordnet ist.

Der Erfindung liegt die Idee zugrunde, eine Hochtemperatursterilisation der Inkubatorkammer nicht "blind" periodisch durchzuführen, sondern unter automatisch erfassbaren Bedingungen dann, wenn eine Kontamination in der Inkubatorkammer entsteht. Dazu soll die Kontamination detektiert werden, mittels Kontaminationsdetektoren (VOC-Sensoren, siehe unten), und vorzugsweise auch der Anwender unmittelbar über eine detektierte Kontamination informiert werden, damit dieser die Hochtemperatursterilisation einleiten kann. Dadurch ergibt sich die Möglichkeit, die Sterilisation unter objektiven Bedingungen bei Bedarf durchzuführen, und somit mit größter Effizienz.

Es ist bekannt, dass durch Kontaminationsvorgänge, insbesondere durch das Wachstum von Bakterien und Mikroorganismen, bestimmte Kontaminationsstoffe in die Umgebung abgegeben werden. Die flüchtigen organischen gasförmigen Stoffwechselprodukte von Mikroorganismen werden als MVOC (engl.: Microbial Volatile Organic Compounds) bezeichnet und können als Indikatoren für mikrobielles Wachstum genutzt werden. Im Rahmen dieser Erfindung werden, in einer Verallgemeinerung des Konzepts der MVOC-Erfassung, alle durch chemische Vorgänge, insbesondere Kontaminationsvorgänge, in die Umgebung bzw. in die Inkubatoratmosphäre abgegebenen Stoffe als VOCs (Volatile Organic Compounds) bezeichnet.

Aus dem Stand der Technik WO 97/08337 A ist eine Einzelgefäßüberwachungsfunktion in Inkubatoren bekannt, die eine Durchgangsöffnung der Inkubatorkammer besitzen, um mittels einer Gasleitung, die in einen oberhalb des Probenvolumens gelegenen Luftüberstandbereich eines in der Inkubatorkammer angeordneten Gefäßes eingeführt ist, Gas aus diesem Luftüberstandbereich zu entnehmen und zu einer außerhalb des Inkubators angeordneten VOC-Sensorvorrichtung zu transportieren. Da sich im Gas des Luftüberstandbereichs eine Gefäßatmosphäre gebildet hat, in der VOC-Spuren aus der im Gefäß enthaltenen Zellkultur angereichert sein können, werden mit dieser Messanordnung gezielt Kontaminationen der im Gefäß enthaltenen Zellkulturen erfasst. Es können aber auf diese Weise keine Kontaminationen erfasst werden, die sich in der Inkubatorkammer selbst gebildet haben, z.B. in einer Wasserwanne der Inkubatorkammer, an Außenseiten von Türdichtungen oder inkubierten Probenbehältern, oder an anderen Stellen, die durch eine kondensationsbedingte Feuchtigkeitsbildung für ein Bakterienwachstum anfällig sind.

Im Rahmen einer der vorliegenden Erfindung zugrunde liegenden Forschungsarbeit wurde eine neue Anordnung entwickelt, bei der die Gasatmosphäre des Innenraums der Inkubatorkammer, in der eine Vielzahl von verschlossenen Zellkulturbehältern angeordnet sein können, mittels einer inkubatoreigenen VOC-Sensoreinrichtung getestet wird, und zwar vorzugsweise als regelmäßig aktive Überwachungsfunktion während der Betriebsdauer des Inkubators. Unabhängig vom Ursprung der Kontamination können die Benutzer auf diese Weise gewarnt werden und eine Sterilisation bzw. Reinigung des Innenraums der Inkubatorkammer kann veranlasst werden. Neben einer Kontamination kann auch -allgemein- eine Anreicherung von VOCs in der Inkubatoratmosphäre gemessen werden, insbesondere um bestimmte biochemische Vorgänge nachzuweisen.
Die Sensoreinrichtung dient der Detektion von Kontamination der Gasatmosphäre des Innenraums. Die Sensoreinrichtung weist vorzugsweise mindestens einen, vorzugsweise genau einen, **VOC-Sensor** zur Detektion von mindestens einer flüchtigen organischen Verbindung (VOC) auf. Dieser VOC-Sensor ist vorzugsweise so gestaltet oder gewählt, dass er für mindestens eine VOC, die durch eine Kontamination freigesetzt wird, empfindlich ist und messen kann, ob sich die Konzentration der VOC in der Gasatmosphäre des Innenraums verändert hat, insbesondere zugenommen hat.

Die Produktion von gasförmigen Stoffwechselprodukten von Mikroorganismen und allen anderen Lebewesen ist eine grundsätzliche Eigenschaft. Die flüchtigen organischen gasförmigen Stoffwechselprodukte von Mikroorganismen werden als MVOC (engl.: Microbial Volatile Organic Compounds) bezeichnet und können als Indikatoren für mikrobielles Wachstum genutzt werden, wobei ihre Bildung hauptsächlich von der Spezies und dem Nährmedium abhängig ist. Da Zellkulturen nicht mikrobielle Organismen darstellen, werden die gasförmigen Stoffwechselprodukte als VOC bezeichnet. Einfachheitshalber werden im Rahmen dieser Patentanmeldung VOCs als auch MVOCs als VOCs bezeichnet. Der Begriff umfasst zudem auch -insbesondere organische- Verbindungen, die z.B. in einem Zeitraum nach der Herstellung aus Kunststoffteilen abdampfen können. VOC bezeichnet nicht das CO2-Gas (Kohlenstoffdioxidgas), das insbesondere ein Bestandteil der Gasatmosphäre der Inkubatorkammer ist. Analog zu den VOCs der Mikroorganismen können die der Zellkulturen ebenfalls als Indikatoren für das Wachstum genutzt werden, da diese Informationen über den metabolischen und physiologischen Zustand der Zellen liefern.

Die Sensoreinrichtung weist vorzugsweise eine Mehrzahl N mit N>1 von VOC-Sensoren, vorzugsweise eine Vielzahl N>=10 von VOC-Sensoren auf. Vorzugsweise ist die Anzahl der VOC-Sensoren der Sensoreinrichtung beschränkt auf eine Maximalzahl M=2000, 100, 50, 25 oder 10. Die VOC-Sensoren können alle typenunterschiedlich sein, wodurch sich ein Spektrum an typenunterschiedlichen Messergebnissen und damit eine bessere Differenzierbarkeit der VOC-Detektion oder einer möglichen VOC-Erkennung bzw. einer möglichen VOC-Klassifizierung ergibt. Es können auch mehrere VOC-Sensoren desselben Typs vorgesehen sein. Beispielsweise ist aus der Biologie bekannt, dass die menschliche Nase ca. 380 unterschiedliche Rezeptortypen verwendet, andere Säugetiere eine deutlich höhere Anzahl, so dass dieser Ansatz als technisch "bewährt" angesehen werden kann.

Vorzugsweise ist eine Sensoreinrichtung als elektronische Nase eingerichtet, bei der eine Mehr- oder Vielzahl von VOC-Sensoren, insbesondere zeitgleich, die Gasatmosphäre des Innenraums messen, insbesondere indem zeitgleich die Anlagerung von VOCs aus der Gasatmosphäre an die Messbereiche der VOC-Sensoren erfasst wird. Insbesondere ist eine elektronische Nase geeignet bzw. dazu eingerichtet, dass gesammelte Messsignale der Mehr- oder Vielzahl N von VOC-Sensoren, insbesondere jedes Messsignal eines jeden der VOC-Sensoren, gemeinsam ausgewertet werden, um eine Kontamination zu detektieren, insbesondere die Art oder eine Klasse einer Kontamination zu erkennen, vorzugsweise auch zu quantifizieren. Eine elektronische Nase ist insbesondere dazu eingerichtet, mindestens zwei in der Gasatmosphäre vorhandene VOCs zu unterscheiden. Eine elektronische Nase ist insbesondere dazu eingerichtet, zwischen verschiedenen Arten oder Klassen von Kontaminationen zu differenzieren. Ferner kann mittels mehrerer VOC-Sensoren eine zuverlässigere und damit auch empfindlichere Detektion von Kontamination erreicht werden als mit einem einzigen VOC-Sensor.

Vorzugsweise weist die Sensoreinrichtung mindestens einen VOC-Sensor auf, der für die Detektion von gasförmigen Stoffwechselprodukten von Mikroorganismen, insbesondere Bakterien, Myoplasmen, Pilzen, Hefen eingerichtet ist. Vorzugsweise weist die Sensoreinrichtung mehrere VOC-Sensoren, insbesondere unterschiedlicher Selektivität der Detektion auf.

Vorzugsweise weist die Sensoreinrichtung mindestens einen VOC-Sensor auf, der für die Detektion von gasförmigen Stoffwechselprodukten von biologischen Zellen eingerichtet ist. Dadurch lassen sich Informationen über den Wachstumsstatus, insbesondere dessen zeitliche Entwicklung, einer Zellkultur gewinnen.

Vorzugsweise weist die Sensoreinrichtung mindestens einen VOC-Sensor auf, der für die Detektion von gasförmigen Stoffen eingerichtet ist, die aus dem Material von Inkubatorbauteilen entweichen, insbesondere in einem bestimmten Zeitraum nach der Herstellung desd Inkubators. Zu diesen gasförmigen Stoffen können Lösungsmittel oder Weichmacher gehören, oder andere Stoffe, die Kunsstoffbauteilen des Inkubators entweichen.

Vorzugsweise weist die Sensoreinrichtung mindestens einen VOC-Sensor auf, der für die Detektion von mindestens einer zu den Alkoholen gehörenden chemischen Verbindung eingerichtet ist, vorzugsweise von mehreren unterschiedlichen, zu den Alkoholen gehörenden chemischen Verbindungen eingerichtet ist. In den dieser Erfindung zugrunde liegenden Forschungsarbeiten wurde festgestellt, dass sich im Rahmen einer Kontaminationsdetektion Alkohole besonders sensibel detektieren lassen. Dies liegt daran, dass Stoffwechselvorgänge von Bakterien, die für die Kontamination von Inkubatoren besonders relevant sind, in besonderem Maße Alkohole erzeugen. Andererseits sind die entsprechenden kommerziell erhältlichen Alkoholsensoren geeignet empfindlich, um bereits geringe Alkoholspuren zu detektieren.

Vorzugsweise weist die Sensoreinrichtung mindestens einen VOC-Sensor auf, der für die Detektion von mindestens einer zu den Aromaten gehörenden chemischen Verbindung eingerichtet ist, vorzugsweise von mehreren unterschiedlichen, zu den Aromaten gehörenden chemischen Verbindungen eingerichtet ist. In den dieser Erfindung zugrunde liegenden Forschungsarbeiten wurde insbesondere auch festgestellt, dass sich im Rahmen einer Kontaminationsdetektion auch Aromaten besonders sensibel detektieren lassen. Auch dies liegt daran, dass Stoffwechselvorgänge von Bakterien, die für die Kontamination von Inkubatoren besonders relevant sind, verschiedene Aromaten erzeugen. Andererseits sind die entsprechenden kommerziell erhältlichen Aromatensensoren geeignet empfindlich, um bereits geringe Aromatenspuren zu detektieren.

Vorzugsweise weist die Sensoreinrichtung mindestens einen VOC-Sensor auf, der für die Detektion von mindestens einer zu den Alkanen gehörenden chemischen Verbindung eingerichtet ist, vorzugsweise von mehreren unterschiedlichen, zu den Alkanen gehörenden chemischen Verbindungen eingerichtet ist. In den dieser Erfindung zugrunde liegenden Forschungsarbeiten wurde insbesondere auch festgestellt, dass sich im Rahmen einer Kontaminationsdetektion auch Alkane besonders sensibel detektieren lassen. Auch dies liegt daran, dass Stoffwechselvorgänge von Bakterien, die für die Kontamination von Inkubatoren besonders relevant sind, verschiedene Alkane erzeugen. Dies gilt insbesondere auch für Stoffwechselvorgänge von Zellen. Andererseits sind die entsprechenden kommerziell erhältlichen Alkansensoren geeignet empfindlich, um bereits geringe Alkanspuren zu detektieren.

Besonders bevorzugt ist der VOC-Sensor eingerichtet zur Erfassung von Alkoholen, und Aromaten und/oder Alkanen.

Vorzugsweise weist die Sensoreinrichtung mindestens einen VOC-Sensor auf, der für die Detektion von mindestens einer der chemischen Verbindungen eingerichtet ist, vorzugsweise von mehreren unterschiedlichen chemischen Verbindungen eingerichtet ist, die der Gruppe von chemischen Verbindungen umfassend {Alkohole, Aromaten, Benzole, Alkylbenzole, Alkane, Alkene, Alkane, Aldehyde, Ester, Ketone, Pyrazole, Oxime, Terpene, Säuren, Carbonsäuren, Heterocyclische Amine und Indolen}. In den dieser Erfindung zugrunde liegenden Forschungsarbeiten wurde insbesondere auch festgestellt, dass sich im Rahmen einer Kontaminationsdetektion diese Stoffe besonders sensibel detektieren lassen. Auch dies liegt daran, dass Stoffwechselvorgänge von Bakterien, die für die Kontamination von Inkubatoren besonders relevant sind, verschiedene solcher Stoffe erzeugen. Dies gilt insbesondere auch für Stoffwechselvorgänge von Zellen. Andererseits sind die entsprechenden kommerziell erhältlichen Sensoren für verschieden Stoffe geeignet empfindlich, um bereits geringe dieser Stoffspuren zu detektieren.

Ein VOC-Sensor ist insbesondere ein chemischer Sensor für die Detektion von VOCs in einer Gasatmosphäre. Die in der Gasatmosphäre detektierten, gleichartigen oder unterschiedlichen VOCs werden vom Sensor detektiert und in ein elektrisches Signal umgewandelt.

Ein VOC-Sensor kann ein Leitfähigkeitssensor sein, der insbesondere eine in Abhängigkeit von mindestens einem VOC veränderliche elektrische Leitfähigkeit misst. Der VOC-Sensor ist besonders bevorzugt ein Metalloxid-Halbleiter (MOX) Gassensor, auch kurz als MOX-Sensor bezeichnet. Solche chemischen Sensoren besitzen eine Detektionsschicht, mithilfe der eine chemische Interaktion in ein elektrisches Signal transformiert werden kann. Sie sind für einen kontinuierlichen Messbetrieb geeignet.

Die Funktion eines MOX-Sensors basiert insbesondere darauf, dass sich abhängig von der Konzentration des Zielgases die elektrische Leitfähigkeit der gassensitiven Metalloxidschicht bzw. des Halbleiters verändert und damit die Anwesenheit als auch Menge des Zielgases bestimmt wird. Typischerweise besteht ein MOX-Sensor aus vier Elementen: Gassensitive Metalloxidschicht, Elektroden, Heizelement und Isolierungsschich. Das Heizelement ist von der gassensitiven Metalloxidschicht und den Kontaktelektroden durch die Isolierungsschicht getrennt. Die gassensitive Metalloxidschicht wird durch das Heizelement erhitzt und Sauerstoffmoleküle aus der Umgebung werden an der Oberfläche der gassensitiven Metalloxidschicht adsorbiert. Die adsorbierten Sauerstoffmoleküle fangen Elektronen von den leitenden Bändern des Halbleiters ein und es bilden sich energetische Barrieren, die so einen Teil des Elektronenflusses im Halbleiter blockieren und somit die elektrische Leitfähigkeit verschlechtern bzw. den Widerstand des Gassensors erhöhen. Sobald reduzierende Gase (Zielgase) präsent sind, reagieren diese mit den gebundenen Sauerstoffmolekülen. Die Sauerstoffmoleküle werden von der Oberfläche der gassensitiven Metalloxidschicht gelöst und die Leitfähigkeit steigt bzw. der Widerstand sinkt.

Ein VOC-Sensor kann ein kapazitativer Sensor sein, der insbesondere eine in Abhängigkeit von mindestens einem VOC veränderliche elektrische Kapazität erfasst. Ein VOC-Sensor kann ein optischer Sensor sein, der insbesondere eine in Abhängigkeit von mindestens einem VOC veränderliche optische Eigenschaft misst, z.B. einen sich ändernden Brechungsindex, eine sich ändernde Lichtintensität, oder ein sich änderndes Lichtspektrum, wobei die Wellenlänge des verwendeten Lichtes nicht beschränkt ist und insbesondere auch Infrarot umfasst. Ein VOC-Sensor kann ein massensensitiver Sensor sein, der insbesondere eine in Abhängigkeit von mindestens einem VOC veränderliche Masse misst, z.B. durch Erfassung einer veränderten Schwingung eines mit dem mindestens einen VOC wechselwirkenden Schwingkörpers.

Die Sensoreinrichtung ist vorzugsweise ein Bestandteil des Inkubators, und ist insbesondere in einem Gehäuse oder Gehäuseteil des Inkubators angeordnet.

Vorzugsweise ist eine elektronische Auswertungseinrichtung vorgesehen, die vorzugsweise eine Datenverarbeitungseinrichtung beinhaltet. Die Auswertungseinrichtung ist dazu eingerichtet, mindestens ein Messsignal, insbesondere Messwert, des mindestens einen VOC-Sensors zu erfassen und insbesondere auszuwerten. Die Auswertungseinrichtung ist vorzugsweise ein Bestandteil des Inkubators, und ist insbesondere in einem Gehäuse oder Gehäuseteil des Inkubators angeordnet. Die Auswertungseinrichtung kann auch Bestandteil der Sensoreinrichtung sein, insbesondere, wenn die letztere als Teil eines Nachrüstsystems für einen Inkubator eingerichtet ist, der insbesondere eine solche Sensoreinrichtung noch nicht aufweist.

Die Auswertungseinrichtung, insbesondere der Inkubator, weist vorzugsweise eine Datenspeichereinrichtung auf, die mit der Datenverarbeitungseinrichtung zum Austausch von Daten verbunden ist.

Die Auswertungseinrichtung, insbesondere die Datenverarbeitungseinrichtung, ist vorzugsweise mit einem Programmcode programmierbar, und insbesondere dazu programmiert, die folgenden Schritte, insbesondere gemäß diesem Programmcode, auszuführen:
- Empfangen mindestens eines Messsignals, insbesondere von Messdaten, mindestens eines VOC-Sensors; insbesondere: Empfangen einer Folge von Messsignalen zeitlich, insbesondere für die Dauer einer Messzeit Δt, nacheinander, die insbesondere den zeitlichen Verlauf der Messung mindestens eines VOC-Sensors bilden;
- Vergleich des mindestens einen Messsignals mit mindestens einem Referenzwert;
- Entscheiden, anhand des Ergebnisses dieses Vergleichs, ob eine Veränderung der VOC-Konzentration in der Gasatmosphäre des Innenraums vorliegt, insbesondere eine Veränderung, die für eine Kontamination des Innenraums charakteristisch ist.

Der genannte mindestens eine Referenzwert kann vorbestimmt sein und kann in der Datenspeichereinrichtung gespeichert sein. Der genannte mindestens eine Referenzwert kann auch ein Startwert sein, der aus einer Messung zu Beginn der Messzeit Δt resultiert, während der der mindestens eine VOC-Sensor die VOC-Moleküle in der Gasatmosphäre des Innenraums erfasst. Der Startwert eines VOC-Sensors kann insbesondere dann erfasst werden, wenn der Messbereich des jeweiligen VOC-Sensors initialisiert wurde, insbesondere indem der Messbereich mit einem Spülgas, z.B. Stickstoff N2, gespült wurde. Dieses Spülen erfolgt vorzugsweise solange, bis der zeitliche Verlauf des Messsignals des mindestens einen VOC-Sensors konstant ist oder einen bekannten Referenzverlauf aufweist, z.B. linear zunehmend oder abnehmend ist.

Der Inkubator, insbesondere die Sensoreinrichtung, weist vorzugsweise eine elektronische Steuereinrichtung auf, die insbesondere eine zweite Datenverarbeitungseinrichtung aufweisen kann oder die die Datenverarbeitungseinrichtung der Sensoreinrichtung verwendet und die dazu programmiert ist, mindestens eine Ventil zu steuern, durch dessen Öffnen ein Spülgas, insbesondere N2, über den Messbereich des mindestens einen VOC-Sensors strömt. Dieses Ventil kann insbesondere in Abhängigkeit von den Messignalen des mindestens einen VOC-Sensors geöffnet und/oder geschlossen werden.

Die Auswertungseinrichtung, insbesondere die Datenverarbeitungseinrichtung, ist vorzugsweise programmierbar, und insbesondere dazu programmiert, die folgenden Schritte auszuführen:
- Empfangen mindestens eines Messwerts, insbesondere von Messdaten, von mindestens zwei, mehreren oder allen einer Mehr- oder Vielzahl von VOC-Sensoren; insbesondere für jeden dieser mindestens zwei VOC-Sensoren;
- insbesondere zeitlich parallel: Empfangen einer Folge von Messwerten mindestens eines VOC-Sensors zeitlich, insbesondere für die Dauer einer Messzeit Δt, nacheinander, die insbesondere den zeitlichen Verlauf der Messung mindestens eines VOC-Sensors bilden;
- Vergleich des mindestens einen Messwerts jedes der mindestens zwei VOC-Sensoren mit mindestens einem Referenzwert;
- Entscheiden, anhand des Ergebnisses dieses Vergleichs, ob eine Veränderung der VOC-Konzentration in der Gasatmosphäre des Innenraums vorliegt, insbesondere eine Veränderung, die für eine Kontamination des Innenraums charakteristisch ist.

Der mindestens eine Referenzwert kann insbesondere dadurch ermittelt werden, dass, in einem Zeitraum vor oder nach der Messung des Gasvolumens der Inkubatoratmosphäre, der mindestens eine Messbereich des mindestens einen VOC-Sensors mit einem Spülgas, insbesondere Stickstoff, gespült wird und die Referenzmessung zur Erfassung eines oder mehrerer Referenzwerte am mit Spülgas gespülten Messbereich durchgeführt wird. Der Vergleich von Messwert und Referenzwert -unter optional zusätzlicher Berücksichtigung eines routinemäßig wählbaren Toleranzwertes, führt zuverlässig zur Detektion von VOCs in der Gasatmosphäre einer Inkubatorkammer.

Wie im Rahmen der dieser Erfindung zugrunde liegenden Forschungsarbeiten herausgefunden wurde, lassen sich insbesondere bakteriell bedingte Anreicherungen von VOCs, also Kontaminationen, in der Gasatmosphäre einer Inkubatorkammer gut nachweisen, da Bakterien aufgrund ihres exponentiellen Wachstums in der entsprechenden Log-Phase einen exponentiell wachsenden Stoffwechsel mit entsprechend exponentiell wachsender VOC-Freisetzung aufweisen. Im Vergleich dazu ist der Ausstoß von VOCs aus normal wachsenden Zellkulturen sehr gering, insofern beispielsweise durch nicht dicht verschlossene Zellkulturbehälter, z.B. Petrischalen, ein Anteil dieser "normalen" VOCs in die Gasatmosphäre der Inkubatorkammer eintritt. Der bakteriell bedingte Stoffwechsel dominiert somit den VOC-bedingten "Geruch" der Gasatmosphäre einer Inkubatorkammer.

Die Auswertungseinrichtung, insbesondere die Datenverarbeitungseinrichtung, ist vorzugsweise programmierbar, und insbesondere dazu programmiert, die folgenden Schritte oder mindestens einen der folgenden Schritte auszuführen:
- optional: Erzeugen von Ergebnisdaten, die die Information darüber enthalten, ob eine Veränderung der VOC-Konzentration in der Gasatmosphäre des Innenraums vorliegt, insbesondere eine Veränderung, die für eine Kontamination des Innenraums charakteristisch ist;
- optional: Ausgabe einer Information an einen Benutzer des Inkubators in Abhängigkeit von den Ergebnisdaten, ob eine Veränderung der VOC-Konzentration in der Gasatmosphäre des Innenraums vorliegt, insbesondere eine Veränderung, die für eine Kontamination des Innenraums charakteristisch ist; die Ausgabe dieser Information kann in Form von visuellen oder akustischen Signalen erfolgen, die mittels einer geeigneten Ausgabeeinrichtung des Inkubators, insbesondere eines Display, und/oder eines Lautsprechers, erzeugt werden. Diese Ausgabe kann insbesondere als Warnsignal ausgestaltet sein, um den Benutzer vor einer bestehenden Kontamination zu warnen;
- optional: Speichern der Ergebnisdaten in der Datenspeichereinrichtung;
- optional: Übertragen der Ergebnisdaten an eine externe Datenverarbeitungseinrichtung, insbesondere an einen PC, ein Smartphone, einen Tabletcomputer, insbesondere mittels einer drahtgebundenen oder drahtlosen Signalverbindung.

Ein VOC-Sensor dient zur Detektion von flüchtigen organischen Verbindungen (VOCs), wobei der VOC-Sensor einen Messbereich aufweist, der in Strömungsverbindung mit dem Atmosphärengas des Innenraums stehend angeordnet ist. Vorzugsweise ist der Messbereich mindestens eines VOC-Sensors oder mehrerer oder aller VOC-Sensoren in der Inkubatorkammer angeordnet. Vorzugsweise ist der Messbereich mindestens eines VOC-Sensors oder mehrerer oder aller VOC-Sensoren in mindestens einer oder mehreren Messkammer(n) angeordnet. Die Innenraumatmosphäre der Messkammer steht vorzugsweise in Strömungsverbindung mit der Gasatmosphäre des Innenraums der Inkubatorkammer. Diese Strömungsverbindung kann gebildet sein, indem die Inkubatorkammer und die Messkammer durch mindestens einen Strömungskanal verbunden sind. Die Messkammer kann aber auch in der Inkubatorkammer angeordnet sein oder der Innenraum der Messkammer kann, ohne dass ein dedizierter Strömungskanal erforderlich wäre, unmittelbar münden in den Innenraum der Inkubatorkammer. Als Messkammer kann auch der Abschnitt eines durchströmten Strömungskanals angesehen werden, entlang dem der mindestens eine Messbereich des mindestens einen VOC-Sensors angeordnet ist. Eine Messkammer kann eine Zuströmöffnung aufweisen, durch die das zu messende Gas in die Messkammer eintritt, und insbesondere eine Abströmöffnung, durch die das zu messende Gas aus der Messkammer austritt.

Die Sensoreinrichtung weist insbesondere mindestens einen Temperatursensor und/oder einen Luftfeuchtesensor und/oder eine Drucksensor auf, der vorzugsweise zur Messung der Gasatmosphäre in der Messkammer angeordnet ist.

Der mindestens eine Messbereich des mindestens einen VOC-Sensors ist vorzugsweise in einem Abschnitt der Messkammer angeordnet, der, betrachtet entlang einer geraden Verbindung zwischen einem Zuströmöffnung und einer Abströmöffnung der Messkammer, zwischen dieser Zuströmöffnung und dieser Abströmöffnung liegt. Der mindestens eine Messbereich des mindestens einen VOC-Sensors ist vorzugsweise parallel einer Strömungsrichtung, insbesondere der Hauptströmungsrichtung in einer Messkammer angeordnet. Diese Strömungsrichtung folgt insbesondere einer Linie, insbesondere einer geraden Linie, welche die Zuströmöffnung und die Abströmöffnung der Messkammer verbindet. Der mindestens eine Messbereich des mindestens einen VOC-Sensors ist vorzugsweise parallel einer Seitenwand der Messkammer angeordnet und/oder bildet eine Seitenwand der Messkammer. Dabei ist eine Seitenwand insbesondere eine Innenwand der Messkammer, an der die Strömung des zu messenden Gases/der zu messenden Atmosphäre entlang strömt. Durch dieser Maßnahmen wird insbesondere erreicht, dass die Konzentration von VOCs aus der Gasatmosphäre entlang des Messbereichs möglichst konstant ist, indem ein durch Abscheidung von VOCs am Messbereich eventuell erschöpfte Gasatmosphäre durch originale Gasatmosphäre ersetzt wird.

Es ist möglich und bevorzugt, dass die Messbereiche mehrerer VOC-Sensoren parallel zueinander und insbesondere parallel zu einer Strömungsrichtung durch die Messkammer angeordnet sind. Es ist möglich und bevorzugt, dass die Messbereiche mehrerer VOC-Sensoren parallel zueinander und/oder insbesondere parallel zu einer Strömungsrichtung durch die Messkammer angeordnet sind. Messbereich können dabei einen Strömungsabschnitt durch die Messkammer umgeben, können insbesondere konzentrisch um diesen Strömungsabschnitt bzw. die Strömungsrichtung angeordnet sein. Messbereiche sind insbesondere so um den Strömungsabschnitt bzw. die Strömungsrichtung angeordnet, dass ein in der Messkammer befindlicher und durch die Messkammer strömender Gasvolumenabschnitt gleichzeitig an mehreren bzw. allen Messbereichen mehrerer VOC-Sensoren anliegt. Dadurch wird insbesondere sicher gestellt, dass an den Messbereichen dieselbe Gaszusammensetzung vorherrscht, und insbesondere nicht etwa ein vorgelagertes Abscheiden von VOCs an einem stromaufwärts gelegenen Sensor dazu führt, dass der stromabwärts gelegene Sensor eine andere Gaszusammensetzung misst.

Ein Strömungskanal ist insbesondere durch mindestens eine Gasleitung gebildet. Deren eines Ende mündet insbesondere in die Inkubatorkammer, und deren anderes Ende insbesondere in die Messkammer.

Mindestens eine Ventileinrichtung kann vorgesehen sein, um gesteuert durch eine elektronische Steuerungseinrichtung des Inkubators oder der Sensoreinrichtung, den Durchgang des mindestens einen Strömungskanals zu öffnen oder zu schließen, insbesondere auch zu drosseln. Die Ventileinrichtung kann insbesondere mindestens ein Wegeventil aufweisen, insbesondere mindestens ein 3/2-Wegeventil. Der Inkubator bzw. die Sensoreinrichtung kann insbesondere ein Spülgasreservoir aufweisen, in dem Spülgas, insbesondere N2 oder ein Edelgas, zum Spülen der Messkammer enthalten ist und das mittels des Ventils an den mindestens einen Strömungskanal angeschlossen ist. Das mindestens eine Wegeventil kann dazu eingerichtet sein, in einer ersten Schaltstellung den Durchgang durch das Ventil zwischen Inkubatorkammer und Messkammer zu öffnen und gleichzeitig den Durchgang durch das Ventil zwischen Spülgasreservoir und Messkammer zu schließen, und in einer zweiten Schaltstellung den Durchgang durch das Ventil zwischen Inkubatorkammer und Messkammer zu schließen und gleichzeitig den Durchgang durch das Ventil zwischen Spülgasreservoir und Messkammer zu öffnen. Anstelle eines Spülgasreservoirs kann auch ein Anschluss zum Zuführen von Spülgas vorgesehen sein, an dem ein Spülgasreservoir anschließbar ist oder ein Spülgasstrom (z.B. Förderung von -insbesondere gefilterter- Umgebungsluft) zuführbar ist.

Vorzugsweise weist der Inkubator bzw. die Sensoreinrichtung eine Gasfördereinrichtung, insbesondere ein Ventilator oder eine Pumpe auf, mittels der Gasatmosphäre aus der Inkubatorkammer durch den Strömungskanal in die Messkammer befördert wird. Die Messkammer weist vorzugsweise einen Strömungskanal, insbesondere mindestens eine Gasleitung, auf, durch den Gasatmosphäre aus der Messkammer aus dieser herausgeführt wird. Insbesondere wird Gasatmosphäre aus der Messkammer in die Umgebung des Inkubators geführt, insbesondere in einen Gehäusebereich des Inkubators, der zur Umgebung offen ist.

Es ist aber auch möglich und bevorzugt, dass ein Strömungskanal vorgesehen ist, der das aus der Messkammer austretende Gas zurück in die Inkubatorkammer führt. Dadurch wird ein Verlust von Inkubationsgasen, insbesondere CO2, verhindert. Vorzugsweise wird das zurückgeführte Gas durch eine Filtereinrichtung geführt, die insbesondere einen HEPA Filter aufweisen kann. Die Filtereinrichtung ist insbesondere dazu eingerichtet, Stoffe und Partikel, die von der Messkammer dem Gas beigefügt wurden, wieder auszufiltern.

Vorzugsweise ist mindestens ein Strömungskanal, insbesondere eine Gasleitung, welche die Inkubatorkammer und die Messkammer stromaufwärts oder stromabwärts der Messkammer verbindet, insbesondere auch mindestens ein Abschnitt mindestens einer Messkammeraußenseite, mit einer thermischen Isoliereinrichtung thermisch isoliert. Die thermische Isoliereinrichtung kann eine Doppelwand aufweisen, oder eine thermisch isolierende Materialschicht, die insbesondere isolirend wirkende Lufteinschlüsse aufweisen kann.

Vorzugsweise ist mindestens ein Strömungskanal, insbesondere eine Gasleitung, welche die Inkubatorkammer und die Messkammer stromaufwärts oder stromabwärts der Messkammer verbindet, in Kontakt mit einem Temperiermittel, insbesondere einer Heizeinrichtung, um den Strömungskanal zu erwärmen, insbesondere auf die Temperatur der Inkubatorkammer oder höher. Insbesondere kann der mindestens eine Strömungskanal an einer Wand der -ohnehin bereits- temperierten Inkubatorkammer entlang geführt sein. Mit solchen Maßnahmen soll verhindert werden, dass Kondensationseffekte auftreten oder dass VOCs temperaturbedingt verändert werden oder ausfallen.

Die Gasatmosphäre des Innenraums des Inkubators weist insbesondere eine andere Zusammensetzung auf als die Atmosphäre in den Zellkulturbehältern, die in dem Innenraum angeordnet sind. Die Atmosphäre in den Zellkulturbehältern ist insbesondere unmittelbar mit flüssigen Wachstumsmedien (Zellmedien) in Kontakt. Insbesondere haben das in die Messkammer eintretende Gas und die Gasatmosphäre des Innenraums dieselbe Zusammensetzung.

Der Messbereich des mindestens einen VOC-Sensors ist vorzugsweise in Strömungsverbindung mit dem Atmosphärengas des Innenraums stehend angeordnet, indem er in der Inkubatorkammer angeordnet ist, wobei in diesem Falle insbesondere keine gesonderte Messkammer notwendig wäre, aber vorgesehen sein kann. Der mindestens eine Messbereich kann insbesondere unmittelbar an den Innenraum der Inkubatorkammer angrenzen und mit der Gasatmosphäre des Innenraums unmittelbar in Kontakt stehen, wobei insbesondere ein Abwärme entwickelnder Bestandteil des mindestens einen VOC-Sensors außerhalb der Inkubatorkammer, und insbesondere in einer Umgebung der Inkubatorkammer angeordnet ist. Diese Umgebung ist insbesondere nicht in Kontakt mit dem Messbereich und ist insbesondere gekühlt, vorzugsweise luftgekühlt.

Es ist insbesondere bevorzugt, dass der mindestens eine VOC-Sensor teilweise und insbesondere nicht vollständig in der Inkubatorkammer und/oder in der Messkammer angeordnet ist. Es ist insbesondere bevorzugt, dass mindestens ein dem Messbereich eines Sensors gegenüberliegender Abschnitt des mindestens einen VOC-Sensor außerhalb der Inkubatorkammer und/oder der Messkammer angeordnet ist/sind. Es ist insbesondere bevorzugt, dass mindestens ein Heizmittel bzw. alle Heizmittel des mindestens einen VOC-Sensor außerhalb der Inkubatorkammer und/oder der Messkammer angeordnet ist/sind. Auf diese Weise ist der elektrische Anschluss des mindestens einen VOC-Sensors an eine elektrische Steuereinrichtung möglich und insbesondere vereinfacht. Zudem ist das Temperieren bzw. Kühlen der dem Messbereich abgewandten Seite, insbesondere der Heizmittel, des mindestens eine VOC-Sensors möglich und insbesondere vereinfacht.

**Mittels** der Gasleitung ist insbesondere Atmosphärengas unmittelbar aus der Inkubatorkammer in den Außenraum des Inkubators transportierbar, bzw. wird in dieser Weise unmittelbar transportiert. Die Sensoreinrichtung weist vorzugsweise eine oder vorzugsweise mehrere Messkammern auf, in der mindestens ein Messbereich mindestens eines VOC-Sensors angeordnet ist. Der am einen Ende in die Inkubatorkammer mündende Strömungskanal kann an einem anderen Ende in eine Messkammer münden oder kann aufgeteilt werden, um mit mehreren Enden in mehrere Messkammern zu münden. Insbesondere kann pro Messkammer eine Anzahl N mit 10>=N>=1 von VOC-Sensoren vorgesehen sein, in welche insbesondere die Gasleitung mündet. Es kann genau ein Messbereich eines VOC-Sensors oder es können genau zwei Messbereiche von VOC-Sensoren in einer Messkammer angeordnet sein. Atmosphärengas aus der Inkubatorkammer ist so insbesondere in mindestens eine- vorzugsweise jede- Messkammer transportierbar und wird - insbesondere gesteuert von einer elektrischen Steuereinrichtung- transportiert.

Vorzugsweise weist eine Messkammer eine Abgasleitung auf, die angeordnet ist, um die Abluft aus der Messkammer in einen Außenraum der Inkubatorkammer bzw. des Inkubators zu befördern.

Vorzugsweise weist die Sensoreinrichtung eine Mehrzahl von VOC-Sensoren auf, deren Messbereiche, insbesondere deren Adsorptionsflächen, in Kontakt mit einem Innenraum der Messkammer angeordnet sind.

Vorzugsweise weist die Sensoreinrichtung eine Mehrzahl von VOC-Sensoren auf, deren Messbereiche, insbesondere deren Adsorptionsflächen zur Adsorption von VOCs an den Messbereich, in Kontakt mit einem Innenraum der Messkammer angeordnet sind, wobei die VOC-Sensoren eine Heizseite aufweisen, die jeweils außerhalb der Messkammer angeordnet ist.

Vorzugsweise weist die Messkammer eine torusförmig ausgebildeten Innenraum auf, insbesondere in Form eines geschlossenen Torus, insbesondere mit mindestens einer Zufluss- und mindestens einer Abflussöffnung. Der Torus kann parallel einer Ebene liegen, die Zuflussöffnung kann im Wesentlichen diesseits der Ebene und insbesondere diesseits des Torus angeordnet sein, und die die Abflussöffnung kann im Wesentlichen jenseits der Ebene und insbesondere jenseits des Torus angeordnet sein. Zu- und Abflussöffnung können aber auch in der Ebene liegen oder diese kreuzen.

Insbesondere kann der Inkubator bzw. die Sensoreinrichtung ein Fördermittel zur Gasförderung aufweisen, mittels der ein im Innenraum der Messkammer befindliches, zuvor der Inkubatorkammer entnommenes, Atmosphärengas zirkuliert werden kann. Dabei kann die Zuflussöffnung und/oder die Abflussöffnung einen -insbesondere von einer elektrischen Steuereinrichtung steuerbaren- Verschluss aufweisen, mit dem die entsprechende Öffnung wahlweise verschließbar ist. Durch Zirkulieren des Gases wird ein Gasaustausch an den Messbereichen/Adsorptionsflächen erzielt, und eine längerfristige Abführung von zu messender Gasatmosphäre aus der Inkubatorkammer kann vermieden werden. Dadurch kann der Verbrauch von Gas, z.B. Co2, und Energie vermieden werden, die zum Wiederherstellen der Inkubatoratmosphäre in der Inkubatorkammer und/oder dem Druckausgleich andernfalls erforderlich sein könnten.

Vorzugsweise ist der Inkubator und/oder dessen elektronische Steuereinrichtung dazu eingerichtet, in Abhängigkeit von dem durch einen Strömungskanal aus der Inkubatorkammer in Richtung einer Messkammer abfließenden Volumenstrom einen entsprechenden zufließenden Volumenstrom mindestens eines Inkubatorgases, insbesondere einer festgelegten Mischung von Inkubatorgasen, z.B. N2 und CO2, zu steuern. Dadurch wird die Inkubatoratmosphäre -auch während einer Messung mittels der Sensoreinrichtung- möglichst unverändert gehalten. Auch die Temperatur der Inkubatoratmosphäre, die sich durch diese Gasab- und zuführung eventuell ändern bzw. sinken kann, kann durch Temperieren mittels der Temperaturregeleinrichtung des Inkubators, insbesondere dessen Temperiereinrichtung(en) und Temperatursensor(en), konstant gehalten werden bzw. nachgeregelt werden, insbesondere auf eine Zieltemperatur, z.B. 37 °C.

Vorzugsweise ist die Sensoreinrichtung als elektronische Nase ausgestaltet. Dazu weist sie insbesondere eine elektronische Steuereinrichtung sowie eine Mehrzahl von vorzugsweise typenunterschiedlichen VOC-Sensoren auf, sowie insbesondere eine Spüleinrichtung, mittels der mindestens eine Messkammer durch ein Spülgas gespült werden kann.

Vorzugsweise weist die elektronische Steuereinrichtung einer als elektronischen Nase ausgestalteten Sensoreinrichtung oder eines Inkubators eine Datenverarbeitungseinrichtung mit mindestens einem Datenspeicher auf, die dazu programmiert ist, mindestens einen, mehrere oder jeden der folgenden Schritte auszuführen. Analog kann das erfindungsgemäße Verfahren diese Schritte aufweisen:
i) Speichern eines Messdatensatzes im dem Datenspeicher, der die, insbesondere zeitabhängig, insbesondere innnerhalb desselben mindestens einen Zeitabschnitts, erfassten, Messwerte der Anzahl N>1 von VOC-Sensoren beinhaltet, wobei ein Messwert für das detektierte Messignal des jeweiligen VOC-Sensors charakteristisch ist, das in Gegenwart eines aus der Inkubatorkammer stammenden und am Messbereich des VOC-Sensors anliegenden, insbesondere vorbeiströmenden, Volumens der Gasatmosphäre erfasst wurde;
ii) Ermitteln von ersten Ergebnismessdaten aus einem Vergleich des Messdatensatzes mit einem Referenzdatensatz, insbesondere unter Verwendung einer Differenz des Messdatensatzes und des Referenzdatensatzes, der die, insbesondere zeitabhängig erfassten, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung stammmenden und am Messbereich des VOC-Sensors anliegenden, insbesondere vorbeiströmenden, Spülgases erfasst wurde;
iii) optional: Erkennen eines charakteristischen Datenmusters in dem die Ergebnismessdaten enthaltenden Ergebnismessdatensatz, wobei das charakteristische Datenmuster ein bestimmtes, im Atmosphärengas nachgewiesenes VOC, insbesondere auch dessen Konzentration oder Menge, repräsentiert.

Das charakteristische Datenmuster in dem die Ergebnismessdaten enthaltenden Ergebnismessdatensatz kann vozugsweise durch folgende Auswertung berücksichtigt werden: eine Kontamination kann insbesondere dann vorliegen, dass die Messwerte einer bestimmten Untermenge von VOC-Sensoren der Sensoreinrichtung -optional unter Berücksichtigung eines Toleranzwertes- von ihrem jeweiligen Referenzwert abweichen, - optional kann ein relativer Grad der Abweichung berücksichtigt werden-, dass aber die Messwerte der anderen VOC-Sensoren der Sensoreinrichtung -optional unter Berücksichtigung eines Toleranzwertes- nicht von ihrem jeweiligen Referenzwert abweichen. Das charakteristische Datenmuster kann zuvor durch Messungen an einem oder mehreren Testgasen mit definiertem VOC-Gehalt ermittelt werden.

Der Schritt iii) kann vorzugsweise beinhalten, dass ein mittels maschinellem Lernen ermittelter Klassifikationsalgorithmus, insbesondere ein künstliches neuronales Netzwerk, zum Klassifizieren des charakteristischen Datenmusters verwendet wird.

Vorzugsweise weist die Steuereinrichtung eine Datenverarbeitungseinrichtung mit mindestens einem Datenspeicher auf, die dazu programmiert ist, mindestens den ersten, oder mehrere oder alle der folgenden Schritte auszuführen. Analog kann das erfindungsgemäße Verfahren diese Schritte aufweisen:
i) Speichern eines Testmessdatensatzes in einem Datenspeicher, der, insbesondere zeitabhängig, insbesondere innnerhalb desselben mindestens einen Zeitabschnitts, erfasste, Testmesswerte der Anzahl N>1 von VOC-Sensoren beinhaltet, wobei ein Testmesswert für das detektierte Messignal des jeweiligen VOC-Sensors charakteristisch ist, das in Gegenwart eines der Messkammer zugeführten und am Messbereich des VOC-Sensors anliegenden, insbesondere vorbeiströmenden, Volumens eines vorbekannten Testgases mit, insbesondere nach Art und/oder Menge, vorbekanntem VOC-Gehalt erfasst wurde;
ii) Ermitteln von zweiten Ergebnismessdaten aus einem Vergleich des Testmessdatensatzes mit einem Referenzdatensatz, insbesondere unter Verwendung einer Differenz des Testmessdatensatzes und des Re-ferenzdatensatzes, der die, insbesondere zeitabhängig, insbesondere innnerhalb desselben mindestens einen Zeitabschnitts, erfassten, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung stammenden und am Messbereich des VOC-Sensors anliegenden, insbesondere vorbeiströmenden, Spülgases erfasst wurde;
iii) Speichern eines die zweiten Ergebnismessdaten enthaltenden zweiten Ergebnismessdatensatzes, der ein nun bekanntes, für das Testgas charakteristisches Datenmuster aufweist.

Mittels den vorstehend genannten Schritten kann insbesondere ein Verfahren realisiert werden, mittels dem die Präsenz bestimmter VOCs in der Gasatmosphäre erkannt werden kann, insbesondere die Konzentration mindestens eines VOC's in der Gasatmosphäre abgeschätzt werden kann.

Vorzugsweise wird ein Schritt iv) ausgeführt, in dem die in iii) erhaltenen zweiten Ergebnismessdaten als gelabelte Daten verwendet werden, um einen lernfähigen Klassifikationsalgorithmus durch maschinelles Lernen zu trainieren, insbesondere ein neuronales Netzwerk, der/das nachfolgend zum Klassifizieren gemessener charakteristischer Datenmusters verwendbar ist. Mittels diesem Schritt kann auch insbesondere ein Verfahren realisiert werden, mittels dem die Präsenz bestimmter VOCs in der Gasatmosphäre erkannt werden kann, insbesondere die Konzentration mindestens eines VOC's in der Gasatmosphäre abgeschätzt werden kann.

Vorzugsweise weist der Inkubator ein Informationsausgabesystem auf, insbesondere ein Display, einen Lautsprecher oder eine Datenschnittstelle zu einem externen datenverarbeitenden Gerät, um in Abhängigkeit von der mittels der Sensoreinrichtung erfassten Detektion von VOCs eine Information über diese Detektion auszugeben, insbesondere um eine Warninformation an einen Benutzer oder ein überwachendes System auszugeben.

Die Erfindung betrifft auch ein Laborüberwachungssystem zur Erfassung der Kontamination mindestens einer Inkubatorkammer, aufweisend
* mindestens einen erfindungsgemäßen Inkubator;
* mindestens ein extern zu dem mindestens einen Inkubator angeordnetes,
datenverarbeitendes Gerät, das insbesondere in einer Datenaustauschverbindung mit dem mindestens einen Inkubator steht, insbesondere über ein Intranet oder das Internet;
wobei das datenverarbeitende Gerät programmiert ist, die von dem mindestens einen Inkubator erhaltenen, mittels der Sensoreinrichtung des Inkubators durch die Detektion ermittelten Messdaten über eine mögliche Kontamination der Inkubatorkammer zu erfassen und in einer Datenspeichereinrichtung zu speichern, insbesondere um diese Messdaten an ein weiteres Gerät zu kommunizieren, insbesondere an einen PC, ein Mobilfunkgerät, ein Smartphone oder einen Tabletcomputer.

Die Erfindung betrifft auch ein Verfahren zur Erfassung der Kontamination in der Inkubatorkammer eines Inkubators, insbesondere eines erfindungsgemäßen Inkubators, aufweisend die Schritte:
* Erfassung von Messdaten mittels einer Sensoreinrichtung, die mindestens einen VOC-Sensor zur Detektion von flüchtigen organischen Verbindungen (VOCs) aufweist, wobei der VOC-Sensor einen Messbereich aufweist, der in Strömungsverbindung mit dem Atmosphärengas des Innenraums stehend angeordnet ist;
* Ermitteln einer möglichen Kontamination der Gasatmosphäre des Innenraums durch Auswertung der Messdaten.

Die Erfindung betrifft auch ein Nachrüstsystem mit einer nachrüstbaren Sensoreinrichtung zur Detektion einer möglichen Kontamination der Gasatmosphäre des Innenraums einer Inkubatorkammer, wobei die Sensoreinrichtung mindestens einen VOC-Sensor (11) zur Detektion von flüchtigen organischen Verbindungen (VOCs) aufweist, wobei der VOC-Sensor einen Messbereich aufweist, der in Strömungsverbindung mit dem Atmosphärengas des Innenraums stehend anordenbar ist, und wobei die Sensoreinrichtung vorzugsweise eine Gasleitung aufweist, die zwischen dem Innenraum und dem Messbereich anordenbar ist, und vorzugsweise eine Pumpe, um ein Volumen der Gasatmosphäre des Innenraums der Inkubatorkammer durch die Gasleitung zum Messbereich zu befördern. Die Erfindung betrifft auch eine solche nachrüstbare Sensoreinrichtung für einen Inkubator. Das Nachrüstsystem bzw. die nachrüstbare Sensoreinrichtung umfasst ferner insbesondere eine elektronische Auswertungseinrichtung, die vorzugsweise eine Datenverarbeitungseinrichtung beinhaltet. Die Auswertungseinrichtung ist vorzugsweise dazu eingerichtet, mindestens ein Messsignal, insbesondere Messwert, des mindestens einen VOC-Sensors zu erfassen und insbesondere auszuwerten. Alternativ und/oder zusätzlich umfasst das Nachrüstsystem einen Programmcode, bei dessen Ausführung eine Datenverarbeitungseinrichtung, insbesondere des Inkubators, den insbesondere einen Messwert des mindestens einen VOC-Sensors erfasst und insbesondere auswertet. Die möglichen Schritte eines Programmcodes wurden bzw. werden hier bereits/noch beschrieben.

Die Erfindung betrifft auch eine Inkubatoranordnung, aufweisend einen Inkubator mit einer Inkubatorkammer und einer nachrüstbaren Sensoreinrichtung wie oben beschrieben, die zur Detektion einer möglichen Kontamination der Gasatmosphäre des Innenraums einer Inkubatorkammer an dem Inkubator oder in der Inkubatorkammer des Inkubators angeordnet ist.

Der Inkubator ist ein Laborgerät bzw. ein Laborinkubator. Ein Inkubator bezeichnet insbesondere ein Laborgerät mit einer Inkubatorkammer, deren Atmosphäre vom Inkubator auf eine vorgegebene Zieltemperatur regelbar ist bzw. geregelt wird. Insbesondere handelt es sich um ein Laborgerät, mit dem kontrollierte Klimabedingungen für verschiedene biologische Entwicklungs- und Wachstumsprozesse geschaffen und erhalten werden können. Der Inkubator kann vorzugsweise ein Shaker, also eine Inkubator mit einer Bewegungseinrichtung zur Bewegung von in der Inkubatorkammer angeordneten Objekten, sein oder diesen beinhalten. Der Inkubator kann eine Zellkultivierunsgvorrichtung sein, ein Microbial incubator (auch ohne CO2). Der Inkubator dient insbesondere der Schaffung und Erhaltung eines Mikroklimas mit geregelten Gas-, und/oder Luftfeuchtigkeits- und/oder Temperatur-Bedingungen in der Inkubatorkammer, wobei diese Behandlung zeitabhängig sein kann. Der Labor-Inkubator, insbesondere eine Behandlungseinrichtung des Labor-Inkubators, kann insbesondere einen Zeitgeber aufweisen, insbesondere eine Zeitschaltuhr, eine Heiz-/Kühleinrichtung und vorzugsweise eine Einstellung für die Regelung eines der Inkubatorkammer zugeführten Austauschgases, eine Einstelleinrichtung für die Zusammensetzung des Gases in der Inkubatorkammer des Inkubators, insbesondere zur Einstellung des CO₂ und/oder des O₂ und/oder des N2-Gehalts Gehalts des Gases und/oder eine Einstelleinrichtung zur Einstellung der Luftfeuchtigkeit in der Inkubatorkammer des Inkubators. Der Inkubator, insbesondere eine Behandlungseinrichtung des Inkubators, weist insbesondere die Inkubatorkammer auf, ferner vorzugsweise eine Regeleinrichtung mit mindestens einem Regelkreis, dem als Stellglied die mindestens eine Heiz-/Kühleinrichtung und als Messglied mindestens eine Temperaturmesseinrichtung zugeordnet sind. Mittels der Regeleinrichtung kann die Temperatur im Inkubator geregelt werden. Je nach Ausführungsform kann darüber auch die Luftfeuchte geregelt werden. Eine mit Wasser gefüllte Wanne in der Inkubatorkammer kann geheizt oder gekühlt werden, um über die Verdunstung die Luftfeuchtigkeit einzustellen. Alternativ und/oder zusätzlich kann ein Wasserverdampfer als Bestandteil des Inkubators vorgesehen sein, mittels dem die Luftfeuchtigkeit in der Atmosphäre der Inkubatorkammer eingestellt wird. CO₂-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen der des mindestens einen Zellkulturbehälters aufweisen.

Eine Sensoranordnung des Inkubators, die insbesondere einer Regeleinrichtung zugeordnet sein kann, weist insbesondere mindestens einen Temperatursensor auf, vorzugsweise eine Vielzahl von Temperatursensoren. Ein Temperatursensor kann beispielsweise ein Pt 100- oder Pt 1000-Temperaturfühler sein. Eine Sensoreinrichtung weist vorzugsweise einen Sensor zur Ermittlung einer relativen Gaskonzentration auf, insbesondere zur Ermittlung des Gehalts an CO₂ und/oder O₂ und/oder N₂. Eine Sensoreinrichtung weist vorzugsweise einen Sensor zur Ermittlung der relativen Luftfeuchtigkeit auf.

Ein Inkubator weist vorzugsweise eine bzw. eine einzige Inkubatorkammer auf. Diese kann in Kompartimente unterteilt sein. Kompartimente können durch -insbesondere gelochte-Lagerplatten getrennt sein, wobei insbesondere ein Gasaustausch zwischen den Kompartimenten ermöglicht ist. Eine Lagerplatte, insbesondere deren untere Seite, kann zum Halten der Sensoreinrichtung oder eines Strömungskanals eingerichtet sein mittels dem Inkubatoratmosphäre in eine Messkammer geführt wird und kann insbesondere eine Halterung für diese Teile aufweisen.

Die Inkubatorkammer weist Kammerwände bzw. Kammerinnenwände und genau eine oder mindestens eine Kammeröffnung auf, über die die Objekte bzw. Zellkulturbehälter im Inneren der Inkubatorkammer platzierbar und entnehmbar sind. Diese Kammeröffnung ist durch ein mit der Inkubatorkammer beweglich verbundenes Verschlusselement verschließbar, insbesondere eine mittels Scharnier an der Inkubatorkammer beweglich montierte Inkubatortüre, insbesondere eine oder mehrere Kammertüren. Ein Inkubator kann eine oder mehrere Innentüren aufweisen, die insbesondere transparent sein können, und kann eine -insbesondere nicht transparente- Außentür aufweisen, welche insbesondere die Inkubatorkammer und gegebenenfalls mindestens einer innere Inkubatortüre, welche die Kammeröffnung verschließt bzw. öffnet, zur Umgebung hin thermisch isoliert.

In der verschlossenen Position der Kammeröffnung ist das Innere (synonym: der Innenraum) der Inkubatorkammer gegen die Umgebung vorzugsweise in der Weise isoliert, so dass im Inneren eine gewünschte, vom Inkubator gesteuerte Atmosphäre einstellbar, insbesondere regelbar ist. Der Begriff "Gasatmosphäre des Innenraums der Inkubatorkammer" bezeichnet nicht den Innenraum von in der Inkubatorkammer angeordneten im Wesentlichen verschlossenen hohlen Objekten, und bezeichnet insbesondere nicht den Behälterinnenraum eines in der Inkubatorkammer angeordneten Behälters, dessen Öffnung verschlossen ist, insbesondere abgedeckt ist, oder nicht gasdicht oder gasdicht verschlossen ist. Dieser Behälterinnenraum, z.B. eines Zellkulturbehälters, weist typischerweise ein flüssiges Medium auf und einen oberhalb dieser Flüssigkeit gelenenen Luftüberstand. In der geöffneten Position der Kammeröffnung ist über diese Öffnung ein Gasaustausch zwischen der Umgebung des Inkubators und dem Inneren der Inkubatorkammer möglich. Die Kammeröffnung befindet sich typischer Weise in einer die Kammeröffnung umlaufenden Frontwand.

Die Inkubatorkammer weist vorzugsweise mehrere Wände bzw. Innenwandflächen auf, die insbesondere einstückig und insbesondere kantenfrei miteinander verbunden sein können. Die Wände bzw. Innenwandflächen sind vorzugsweise im Wesentlichen planar geformt, können aber auch alle oder zum Teil eine geschwungene Form aufweisen. Die Inkubatorkammer ist vorzugsweise quaderartig geformt, kann aber auch anders geformt sein, z.B. sphärisch, ellipsoid, polyederförmig. Die Wände bzw. Innenwandflächen sind vorzugsweise aus einem korrosionsarmen Material gefertigt, insbesondere Edelstahl, Kupfer, Messing, oder ein Kunststoff, insbesondere ein Verbundkunststoff. Dadurch wird die Reinigung/ Sterilisation des Kammerinneren erleichtert. Unabhängig von der Kammeröffnung, die dem Bestücken / Entnehmen von Objekten bzw. Zellkulturbehältern dient, kann die Inkubatorkammer mindestens einen Port zum Durchführen einer entsprechend dimensionierten Vorrichtung bzw. einer Gasleitung und/oder einer Kabelverbindung aus dem Inneren der Inkubatorkammer an deren Außenseite oder in die Umgebung des Inkubators aufweisen. Ein Port beinhaltet insbesondere eine Öffnung in einer Kammerwand der Inkubatorkammer, zum Durchführen einer entsprechend dimensionierten Vorrichtung bzw. einer Gasleitung und/oder einer Kabelverbindung aus dem Inneren der Inkubatorkammer an deren Außenseite oder in die Umgebung des Inkubators

Eine typische Größe des Inneren einer Inkubatorkammer liegt zwischen 50 und 400 Litern.

Der Inkubator kann genau eine Inkubatorkammer aufweisen, kann aber auch mehrere Inkubatorkammern aufweisen, deren Atmosphäre (Temperatur, relative Gaskonzentration, Luftfeuchte) insbesondere individuell oder gesammelt einstellbar sein kann. Ein Inkubator kann mehrere Inkubatorkammern aufweisen, die jeweils eine eigene Kammeröffnung und eine eigene Kammertüre zum Verschließen der Kammeröffnung aufweisen können, insbesondere einen eigenen Port. Es können mehrere Sensoreinrichtungen vorgesehen sein, insbesondere ein pro Kammer.

Der Inkubator kann ein Gehäuse aufweisen, dass die Inkubatorkammer teilweise oder vollständig umgibt. Das Gehäuse kann im Wesentlichen quaderförmig ausgebildet sein, und kann insbesondere derart ausgebildet sein, dass der Inkubator stapelbar ist. Die Sensoreinrichtung und/oder der mindestens eine VOC-Sensor ist vorzugsweis in dem Gehäuse, oder einem Gehäuseabschnitt angeordnet, insbesondere unbeweglich mit diesem verbunden. Auf diese Weise bildet die Sensoreinrichtung einen integralen Bestandteil des Inkubators. Sie kann aber auch als Modul, insbesondere als Teil eines Nachrüstsystems im Gehäuse anordenbar sein, insbesondere unbeweglich mit diesem verbindbar sein.

Ein Lagerbereich des Inkubators ist insbesondere durch eine Lagerplatte, insbesondere einen Regalblecheinsatz realisiert, die/der insbesondere aus Edelstahl oder Kuper oder ähnlichem bestehen kann oder dieses Material aufweist. Eine Lagerplatte dient als Bodenplatte, insbesondere als Zwischenbodenplatte. Die Lagerplatte kann der Inkubatorkammer entnehmbar sein ("Lagerplatteneinsatz") oder kann fest eingesetzt mit dieser verbunden sein. Die Inkubatorkammer kann Halteabschnitte oder einen Haltrahmen zum Halten eines oder mehrerer Lagerplatteneinsätze oder einsetzbarer Instrumente aufweisen. Eine Lagerplatte kann an ihrer Unterseite zum Halten einer Sensoreinrichtung oder mindestens einer Gasleitung eingerichtet sein, insbesondere eine Halterung für diese Sensoreinrichtung oder Gasleitung aufweisen. Alternativ oder zusätzlich kann mindestens eine der Innenwände der Inkubatorkammer zum Halten eines oder mehrerer Lagerplatteneinsätze oder einsetzbarer Instrumente eingerichtet sein. Dazu kann eine in die Wand integrierte Haltestruktur vorgesehen sein, insbesondere ein oder mehrere Vorsprünge, Rinnen oder Stege. Eine Lagerplatte vergrößert die verfügbare Lagerfläche in der Inkubatorkammer.

Ein Halterahmen für die mindestens eine Lagerplatte ist ebenfalls vorzugsweise aus einem nicht-korrosiven Material gefertigt, vorzugsweise Edelstahl. Der Halterahmen ist vorzugsweise als stehendes Objekt ausgelegt, indem er mindestens einen Sockelabschnitt aufweist, der auf der Bodenwand der Inkubatorkammer ruht. Er kann sich aber auch an den Seitenwänden der Inkubatorkammer abstützen und/oder an der Deckenwand der Inkubatorkammer aufgehängt sein.

Eine Lagerplatte erstreckt sich vorzugsweise - und insbesondere im Wesentlichen vollständig- über einen horizontalen Querschnitt der Inkubatorkammer.

Vorzugsweise weist der Inkubator eine Behandlungseinrichtung zur Behandlung des mindestens einen Zellkulturbehälters auf. Der Begriff "Behandlung" bedeutet insbesondere, dass ein Objekt, insbesondere eine Zellkultur oder ein Zellkulturbehältnis bewegt, und/oder transportiert und/oder untersucht und/oder verändert wird, insbesondere physikalisch, chemisch, biochemisch oder auf andere Weise verändert wird.

Eine Behandlungseinrichtung kann eine Bewegungseinrichtung sein, mittels der das Zellmedium in mindestens einem Zellkulturbehälter in Bewegung gehalten wird, vorzugsweise über ein Bewegungsprogramm, das vom Steuerprogramm gesteuert wird. Eine Bewegungseinrichtung kann eine Schüttel- oder Schwenkeinrichtung sein. Eine Bewegungseinrichtung weist vorzugsweise eine Trägereinrichtung auf, insbesondere eine Platte, auf der ein oder mehrere Zellkulturbehälter abgestellt und/oder fixiert werden. Eine Bewegungseinrichtung weist vorzugsweise eine Antriebseinrichtung auf, insbesondere im Fall einer Schütteleinrichtung beispielsweise einen Oszillatorantrieb, mittels dem das gewünschte Bewegungsprogramm umgesetzt wird. Die Gestaltung des Bewegungsprogramms kann vom Wachstumsstadium der Zellen einer Zellkultur abhängen und kann von der Zellart, insbesondere einer Zelllinie abhängen. Die Gestaltung und/oder Steuerung der Behandlung, insbesondere des Bewegungsprogramms, kann von den Zellüberwachungsdaten abhängen. Die Eine Behandlungseinrichtung kann eine Schwenkeinrichtung sein, mittels der mindestens ein Zellkulturbehälter geschwenkt wird. Die Bestandteile der Schwenkeinrichtung können denen der Schütteleinrichtung entsprechen, sind aber für eine Schwenkbewegung eingerichtet.

Eine Behandlungseinrichtung kann auch eine Transporteinrichtung sein, mittels der mindestens ein Zellkulturbehälter in der Inkubatorkammer transportierbar ist. Die Transporteinrichtung kann eine Lifteinrichtung sein, aufweisend eine Trägereinrichtung, auf der der mindestens eine Zellkulturbehälter platzierbar ist. Die Lifteinrichtung weist vorzugsweise einen Bewegungsmechanismus und/oder einen elektrisch ansteuerbaren Antriebsmechanismus zum Antreiben des Bewegungsmechanismus auf. Die Transporteinrichtung kann ferner einen beweglichen und elektrisch ansteuerbaren Greifarm zum Greifen und Halten mindestens eines Zellkulturbehälters sein. Die Transporteinrichtung kann ein Förderband zum Bewegen des mindestens einen darauf platzierten Zellkulturbehälters aufweisen. Durch den Transport kann der mindestens eine Zellkulturbehälter in der Inkubatorkammer bewegt werden, insbesondere zu einer Bearbeitungsposition in einer Bearbeitungsstation in der Inkubatorkammer, und von dieser Bearbeitungsposition weg. Die Steuereinrichtung kann dazu eingerichtet sein, die Transporteinrichtung in Abhängigkeit von Ergebnisdaten zu steuern, die von einer Auswertungseinrichtung aus den Messwerten der Sensoreinrichtung gewonnen wurden.

Die Sensoreinrichtung, insbesondere eine Gasleitung, kann zudem an einer in der Inkubatorkammer befindlichen Transporteinrichtung befestigbar sein bzw. befestigt sein. Die Sensoreinrichtung kann an einem Positioniermechanismus befestigbar sein oder befestigt sein, mittels dem die Sensoreinrichtung in der Inkubatorkammer bewegbar und positionierbar ist. Der Positioniermechanismus kann einen beweglichen Roboterarm beinhalten und ist vorzugsweise elektrisch steuerbar, insbesondere durch ein Steuerprogramm der Steuereinrichtung. Auf diese Weise kann eine VOC-Konzentration an unterschiedlichen Orten in der Inkubatorkammer nacheinander mit einer oder mit wenigen Sensoreinrichtungen nacheinander gemessen werden. Der Positioniermechanismus kann als in die Inkubatorkammer einsetzbares Bauteil ausgebildet sein. Die Energiezufuhr dieses Bauteils kann über eine Kabelverbindung mit dem Inkubator erfolgen, vorzugsweise über ein durch eine Wandöffnung, z.B. einen Port, oder über eine solche Kabelverbindung zu einer externen Spannungsquelle. Die Steuereinrichtung kann dazu eingerichtet sein, den Positioniermechanismus in Abhängigkeit von Ergebnisdaten zu steuern, die von einer Auswertungseinrichtung aus den Messwerten der Sensoreinrichtung gewonnen wurden.

Als Behandlungseinrichtung lässt sich auch die Temperiereinrichtung der Inkubatorkammer verstehen, mit der die Atmosphäre im Inneren der Inkubatorkammer auf den gewünschten Wert, insbesondere 37°C geregelt wird. Der Begriff Temperieren bezieht sich auf das Erhöhen und Senken der Atmosphärentemperatur durch Heizen und Kühlen. Vorzugsweise wird die Temperatur im Inneren über eine Temperaturänderung der Wände des Inkubators eingestellt. Temperatursensoren der entsprechenden Temperaturregeleinrichtung sind an wenigstens einer Position im Inneren und / oder außerhalb der Inkubatorkammer, insbesondere an einer Wand der Inkubatorkammer verteilt.

Der Inkubator weist vorzugsweise eine Benutzerschnittstelleneinrichtung auf, über die der Benutzer Daten an die Datenverarbeitungseinrichtung bzw. die Steuereinrichtung eingeben kann, und/oder über die Informationen an den Benutzer ausgegeben werden können. Vorzugsweise ist der Inkubator bzw. diese Benutzerschnittstelleneinrichtung dazu eingerichtet, dass der Benutzer eine von den Messwerten der Sensoreinrichtung, insbesondere den Ergebnisdaten einer Auswertungseinrichtung, abhängige Information erhalten kann. Vorzugsweise ist der Inkubator bzw. diese Benutzerschnittstelleneinrichtung dazu eingerichtet, dass der Benutzer mindestens einen Betriebsparameter zum Betreiben des Inkubators oder der Sensoreinrichtung an dieser Benutzerschnittstelleneinrichtung eingeben kann und/oder entsprechende Informationen von dieser erhalten kann. Auf diese Weise kann eine einzige Benutzerschnittstelleneinrichtung vom Benutzer dazu verwendet werden, um den Inkubator und auch die mindestens eine Sensoreinrichtung zu beeinflussen, oder zu steuern, oder von dieser Informationen zu erhalten. Insbesondere kann die Sensoreinrichtung dazu eingerichtet sein, dem Benutzer auf eine mittels der Benutzerschnittstelleneinrichtung des Inkubators erfolgte Abfrage des Benutzers Messwerte oder Ergebnisdaten anzuzeigen, oder eine aus Messwerten abgeleitete statistische Information, und/oder einen Zeitverlauf von Messwerten oder Ergebnisdaten.

Eine gerätegesteuerte Behandlung des Inkubators ist vorzugsweise eine programmgesteuerte Behandlung, also eine durch ein Programm gesteuerte Behandlung. Unter einer programmgesteuerten Behandlung einer Probe ist zu verstehen, dass der Vorgang der Behandlung im Wesentlichen durch Abarbeiten einer Mehrzahl oder Vielzahl von Programmschritten erfolgt. Vorzugsweise erfolgt die programmgesteuerte Behandlung unter Verwendung mindestens eines Programmparameters, insbesondere mindestens eines vom Benutzer gewählten Programmparameters. Ein von einem Benutzer gewählter Parameter wird auch als Nutzerparameter bezeichnet. Die programmgesteuerte Behandlung erfolgt vorzugsweise mittels der digitalen Datenverarbeitungseinrichtung, die insbesondere Bestandteil der Steuereinrichtung ist. Die Datenverarbeitungseinrichtung kann mindestens einen Prozessor, d.h. eine CPU aufweisen, und/oder mindestens einen Mikroprozessor aufweisen. Die programmgesteuerte Behandlung wird vorzugsweise entsprechend den Vorgaben eines Programms gesteuert und/oder durchgeführt, insbesondere eines Steuerprogramms. Insbesondere ist bei einer programmgesteuerten Behandlung zumindest nach Erfassung der benutzerseitig erforderlichen Programmparameter im Wesentlichen keine Benutzertätigkeit erforderlich. Eine gerätegesteuerte Behandlung des Inkubators kann insbesondere in Abhängigkeit von Messwerten oder Ergebnisdaten der mindestens einen Sensoreinrichtung erfolgen.

Unter einem Programmparameter wird eine Variable verstanden, die innerhalb eines Programms oder Unterprogramms, für mindestens eine Durchführung (Aufruf) des Programms oder Unterprogramms gültig, in vorbestimmter Weise eingestellt werden kann. Der Programmparameter wird, z.B. vom Benutzer, festgelegt und steuert das Programm oder Unterprogramm und bewirkt eine Datenausgabe in Abhängigkeit von diesem Programmparameter. Insbesondere beeinflusst und/oder steuert der Programmparameter und/oder steuern die vom Programm ausgegebenen Daten die Steuerung des Geräts, insbesondere die Steuerung der Behandlung mittels der mindestens einen Behandlungseinrichtung.

Unter einem Programm wird insbesondere ein Computerprogramm verstanden. Ein Programm ist eine Folge von Anweisungen, insbesondere bestehend aus Deklarationen und Instruktionen, um auf einer digitalen Datenverarbeitungseinrichtung eine bestimmte Funktionalität, Aufgaben- oder Problemstellung bearbeiten und/oder lösen zu können. Ein Programm liegt in der Regel als Software vor, die mit einer Datenverarbeitungseinrichtung verwendet wird. Das Programm kann insbesondere als Firmware vorliegen, im Fall der vorliegenden Erfindung insbesondere als Firmware der Steuereinrichtung des Inkubators oder des Systems. Das Programm liegt meist auf einem Datenträger als ausführbare Programmdatei, häufig im sogenannten Maschinencode vor, die zur Ausführung in den Arbeitsspeicher des Rechners der Datenverarbeitungseinrichtung geladen wird. Das Programm wird als Abfolge von Maschinen-, d. h. Prozessorbefehlen von dem/den Prozessoren des Computers verarbeitet und damit ausgeführt. Unter 'Computerprogramm' wird insbesondere auch der Quelltext des Programms verstanden, aus dem im Verlauf der Steuerung des Laborgerätes der ausführbare Code entstehen kann.

Eine Benutzerschnittstelleneinrichtung kann Bestandteil eines Inkubators sein, oder ein Modul. Eine Benutzerschnittstelleneinrichtung weist jeweils vorzugsweise auf: eine Steuereinrichtung für die Benutzerschnittstelleneinrichtung; eine Kommunikationseinrichtung zur Herstellung einer Datenverbindung mit einem Laborgerät, insbesondere einem Inkubator, über eine Schnittstelleneinrichtung desselben; eine Eingabeeinrichtung zur Erfassung von Benutzereingaben eines Benutzers; eine Ausgabeeinrichtung, insbesondere eine Anzeige und/oder ein Display, zur Ausgabe von Informationen an den Benutzer, insbesondere ein berührungsempfindliches Display. Dabei ist die Steuereinrichtung der Benutzerschnittstelleneinrichtung vorzugsweise dazu eingerichtet, über die Datenverbindung Daten mit der Steuereinrichtung des Inkubators auszutauschen.

Ein in der Inkubatorkammer lagerbares Objekt ist insbesondere ein Zellkulturbehälter. Ein Zellkulturbehälter ist insbesondere transparent. Er ist insbesondere aus Kunststoff gefertigt, insbesondere PE oder PS gefertigt und weist insbesondere eine planare Bodenplatte auf, welche die Wachstumsfläche der Zellen bildet. Diese kann eine Oberflächenbehandlung zur Förderung der Adhärenz von Zellen aufweisen. Der Zellkulturbehälter kann mit einer PE-Kappe oder Gasaustauschkappe, insbesondere einem Deckel mit optional enthaltenem Filter, verschließbar oder verschlossen sein bzw. versehen sein. Der Zellkulturbehälter ist insbesondere stapelbar. Geeignet ist insbesondere eine Eppendorf Zellkulturflasche.

Der Inkubator weist vorzugsweise eine elektrische Steuereinrichtung (synonym: Steuerungseinrichtung) auf, die insbesondere eine Regeleinrichtung beinhalten kann. Eine Datenverarbeitungseinrichtung ist vorzugsweise Bestandteil einer Steuereinrichtung des Inkubators, die Funktionen des Inkubators steuert. Die Funktionen der Steuereinrichtung sind insbesondere durch elektronische Schaltkreise implementiert. Die Steuereinrichtung kann einen Mikroprozessor aufweisen, der die Datenverarbeitungseinrichtung beinhalten kann. Die Steuereinrichtung und/oder die Datenverarbeitungseinrichtung ist vorzugsweise zur Durchführung eines Steuerungsverfahrens ausgebildet, das auch als Steuerungssoftware oder Steuerungsprogramm bezeichnet wird. Die Funktionen des Inkubators und/oder der Steuereinrichtung und/oder der Auswertungseinrichtung können in Verfahrensschritten beschrieben werden. Sie können als Bestandteile eines Computerprogramm(code)s des Steuerungsprogramms realisiert sein, insbesondere als Unterprogramme des Steuerungsprogramms.

Eine Steuereinrichtung weist im Rahmen der vorliegenden Erfindung generell insbesondere die Datenverarbeitungseinrichtung, insbesondere eine Recheneinheit (CPU) zum Verarbeiten von Daten und/oder einen Mikroprozessor auf oder ist die Datenverarbeitungseinrichtung. Die Datenverarbeitungseinrichtung der Steuereinrichtung des Inkubators ist vorzugsweise auch zum Steuern eines Behandlungsprozesses und/oder von individuellen Behandlungen eingerichtet, die von einer oder mehreren insbesondere optionalen Behandlungseinrichtungen des Inkubators durchgeführt werden.

Die Datenverarbeitungseinrichtung ist alternativ vorzugsweise eine außerhalb des Inkubators und getrennt von diesem angeordnete Vorrichtung, auch als externe Vorrichtung bzw. externe Datenverarbeitungseinrichtung bezeichnet. Die Datenverarbeitungseinrichtung und der Inkubator stehen vorzugsweise in einer Datenverbindung und sind vorzugsweise Bestandteile eines Netzwerks zum Datenaustausch. Die Datenverarbeitungseinrichtung und der Inkubator sind in diesem Fall insbesondere Bestandteile eines erfindungsgemäßen Laborüberwachungssystems zur Erfassung der Anreicherung von VOCs, insbesondere zur Erfassung der Kontamination.

Der Inkubator weist insbesondere eine Regeleinrichtung mit mindestens einem Regelkreis, dem als Stellglied die mindestens eine Temperiereinrichtung und als Messglied mindestens ein Temperatursensor zugeordnet sind. Je nach Ausführungsform kann darüber auch die Luftfeuchte geregelt werden, obwohl die Luftfeuchtigkeit selber nicht durch einen Luftfeuchte-Sensor (rH-Sensor) gemessen wird und die Luftfeuchtigkeit nicht Eingangsgröße des Regelkreises ist. Eine mit Wasser gefüllte Wanne in der Inkubatorkammer kann geheizt oder gekühlt werden, um über die Verdunstung die Luftfeuchtigkeit einzustellen. CO2-Inkubatoren dienen insbesondere der Kultivierung tierischer bzw. humaner Zellen. Inkubatoren können Wendevorrichtungen zum Wenden des mindestens einen Zellkulturbehälters und/oder eine Schütteleinrichtung zum Schütteln bzw. Bewegen der des mindestens einen Zellkulturbehälters aufweisen.

Es ist bei der Anordnung einer Sensoreinrichtung an oder in der Inkubatorkammer zu beachten zu beachten, dass der Betrieb von elektrischen Einrichtungen innerhalb der Inkubatorkammer zu Abwärme führt, welche die Kammeratmosphäre in inakzeptablem Maße erwärmen kann. Die Steuereinrichtung des Inkubators kann deshalb insbesondere dazu eingerichtet sein, den Betrieb der elektrischen Einrichtungen, insbesondere der mindestens einen Sensoreinrichtung innerhalb oder an der Inkubatorkammer in Abhängigkeit von mittels Temperatursensoren erfassten Temperaturen der Kammeratmosphäre zu steuern. Die Steuereinrichtung des Inkubators kann insbesondere dazu eingerichtet sein, die Temperierung der Kammeratmosphäre mittels der mindestens einen Temperiereinrichtung und den Betrieb der elektrischen Einrichtungen innerhalb der Inkubatorkammer in Abhängigkeit voneinander zu steuern, um die unerwünschte Erwärmung der Kammeratmosphäre zu kompensieren.

Die Steuerungseinrichtung kann dazu eingerichtet sein, dass ein Programmparameter oder ein Steuerungsparameter des Inkubators automatisch in Abhängigkeit von anderen Daten gewählt wird. Eine von einem Steuerungsparameter gesteuerte Behandlung der mindestens einen Zellkultur in mindestens einem Zellkulturbehälter entspricht bei einem Inkubator insbesondere einer Klimabehandlung, der die mindestens eine Zellkultur unterzogen wird. Mögliche Parameter, insbesondere Programmparameter, insbesondere Nutzerparameter, die zur Beeinflussung einer Klimabehandlung verwendet werden, definieren insbesondere die Temperatur des Inkubatorraums, in dem die mindestens eine Probe inkubiert wird, die relative Gaskonzentration von O2- und/oder CO2 und/oder N2 im Inkubationsinnenraum, die Luftfeuchtigkeit im Inkubationsinnenraum und/oder mindestens einen Ablaufparameter, der den Ablauf, insbesondere die Reihenfolge, eines aus mehreren Schritten bestehenden Inkubationsbehandlungsprogramms beeinflusst oder definiert.

Die Temperiereinrichtung kann eine kombinierte Heiz- / Kühleinrichtung sein. Sie ist vorzugsweise nur eine Heizeinrichtung. Diese kann insbesondere die Wärme über einen elektrischen Widerstandsdraht erzeugen.

Zu den vom Inkubator gesteuerten Vorgängen gehören zudem alle Temperierschritte, die den physikalischen Zustand "Temperatur der Inkubatoratmosphäre" beeinflussen, insbesondere auch Schritte einer optionalen automatischen Sterilisation mittels Hochtemperaturphasen bei ca. 100 °C bis 120 °C oder bis 180°C oder 200°C, die bei leerer Inkubatorkammer durchgeführt werden.

Zu den vom Inkubator gesteuerten Vorgängen können ferner Gasaustauschvorgänge gehören, bei denen gemäß einem Volumenstrom mit einem vorbestimmten Volumen pro Zeit Teile der Inkubatoratmosphäre ausgetauscht werden, um insbesondere als Bestandteil eines Regelvorgangs die gewünschte Gaszusammensetzung der Inkubatoratmosphäre einzustellen, insbesondere wenn sich diese nach einem Öffnen der Inkubatortüre in unerwünschter Weise geändert hat oder ein Absaugen von Gasatmosphäre der Inkubatorkammer in einer Messkammer die Kammeratmosphäre, insbesondere den Kammerdruck, unerwünscht ändert. Der entsprechende zu messende physikalische Zustand ist in diesem Fall die relative Gaskonzentration oder die relative Luftfeuchtigkeit. Der Inkubator bzw. dessen Steuereinrichtung ist vorzugsweise dazu eingerichtet, die zu einem bestimmten Zeitpunkt als Sollwert eines Temperaturregelkreises angelegte die relative Gaskonzentration oder den infolge der relativen Gaskonzentration detektierten Gaswerte, insbesondere CO2 und/oder O2-Werte, zu regeln. Zu dieser Gasregelung lässt sich auch die Regelung der Luftfeuchtigkeit rechnen, die mittels eines Luftfeuchtesensors einer Sensoranordnung erfolgt, insbesondere mittels eines Sensors zur Messung der relativen Luftfeuchtigkeit.

Zu den vom Inkubator selbst gesteuerten Vorgängen können ferner Gasbewegungsvorgänge gehören, bei denen beispielsweise gemäß einem Volumenstrom mit einem vorbestimmten Volumen pro Zeit und insbesondere auch in einer oder mehrere variabler oder konstanter Strömungsrichtungen in der Inkubatorkammer Teile der Inkubatoratmosphäre bewegt werden. Dies kann insbesondere zu einer gleichmäßigeren Atmosphäre innerhalb der Inkubatorkammer führen, um insbesondere unterschiedlich positionierte Zellkulturbehälter im zeitlichen Mittel mit derselben Atmosphäre zu beaufschlagen.

Weitere bevorzugte Ausgestaltungen des erfindungsgemäßen Inkubators, der Sensoreinrichtung und des Verfahrens, ergeben sich aus der nachfolgenden Beschreibung der Ausführungsbeispiele in Zusammenhang mit den Figuren und deren Beschreibung. Gleiche Bauteile der Ausführungsbeispiele werden im Wesentlichen durch gleiche Bezugszeichen gekennzeichnet, falls dies nicht anders beschrieben wird oder sich nicht anders aus dem Kontext ergibt. Es zeigen:
Fig. 1a zeigt eine perspektivische seitlich-frontale Ansicht eines erfindungsgemäßen Inkubators gemäß Ausführungsbeispiel, im geschlossenen Zustand der Gehäusetüre.
Fig. 1b zeigt eine perspektivische seitlich-rückwärtige Ansicht des Inkubators der Fig. 1a.
Fig. 1c zeigt eine perspektivische seitlich-frontale Ansicht des Inkubators der Fig. 1a, im geöffneten Zustand der Gehäusetüre.
Fig. 2 zeigt eine perspektivische seitlich-frontale Ansicht des Inkubators der Fig. 1a, mit ausgeblendeter Gehäusetüre und in einem Querschnitt entlang einer Ebene, die parallel zur Seitenwand und zentral durch den Inkubator verläuft.
Fig. 3a bis 3k zeigen jeweils einen anderen Inkubator gemäß einem jeweils bevorzugten Ausführungsbeispiel der Erfindung.
Fig. 4a zeigt eine Sensoreinrichtung mit Messkammer gemäß einem Ausführungsbeispiel der Erfindung, die insbesondere im Inkubator 1 der Figure 1a bis 2 verwendet werden kann.
Fig. 4b zeigt die eine Sensoreinrichtung mit Messkammer der Fig. 6a in einer Schnittansicht, ohne eingesetzte VOC-Sensoren und ohne deren Kabel.
In Fig 5 ist: der schematische Aufbau der zu Fig. 4a, 4b zuordenbaren Sensoreinrichtung gezeigt und deren Verbindung zu einer Steuereinrichtung des Inkubators.
Fig. 6 zeigt den schematischer Aufbau eines MOX-Sensors, der in einer erfindungsgemäßen Sensoreinrichtung verwendbar ist.
Fig. 7 zeigt schematisch die Gassteuerung bei einer VOC-Messung, die von den in den Fig. 4a bis 6 gezeigten Komponenten ausführbar ist.
Fig. 8 zeigt Diagramme mit Messwerte zu einer Messung von VOCs aus DH5□, die in Zellkulturflaschen in der Inkubatorkammer des erfindungsgemäßen Inkubators wuchsen.
Fig. 9 zeigt Diagramme mit Messwerte zu einer Messung von VOCs aus CHO-CD-Medium, die in Zellkulturflaschen in der Inkubatorkammer des erfindungsgemäßen Inkubators wuchsen.
Fig. 10 zeigt Diagramme mit Messwerte zu einer Messung von VOCs aus DH5a in CHO-CD-Medium, die in Zellkulturflaschen in der Inkubatorkammer des erfindungsgemäßen Inkubators wuchsen.
Fig. 11 zeigt Diagramme mit Messwerten zu einer Messung von VOCs aus DH5□ und CHO-S in CHO-CD-Medium, die in Zellkulturflaschen in der Inkubatorkammer des erfindungsgemäßen Inkubators wuchsen.
Fig. 12 zeigt Diagramme mit Messwerten zu einer Messung von VOCs aus DH5a in CHO-CD-Medium, die in Zellkulturflaschen in der Inkubatorkammer des erfindungsgemäßen Inkubators wuchsen.. Die aufgenommen Messsignale der Gassensoren werden über die Zeit aufgetragen und der ggf. vorliegende Alarmzeitpunkt markiert (vertikale schwarze Markierung).

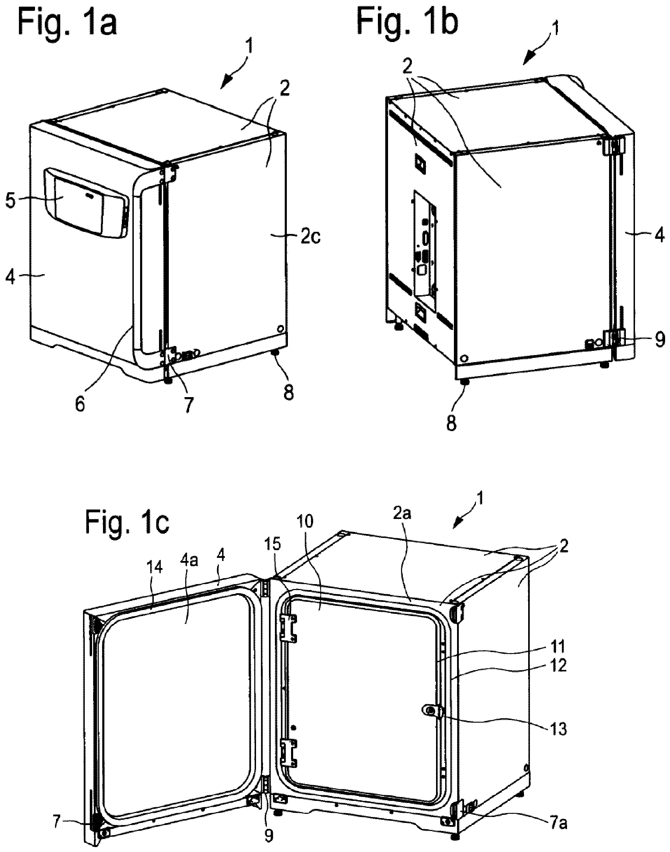

Fig. 1a zeigt die den Inkubator 1 für das Wachstum von Zellkulturen, hier ein als CO2-Inkubator ausgebildeten Labortemperierschrank für das Wachstum von eukaryotischen Zellen. Der Inkubator 1 hat ein Gehäuse 2 mit einem von mindestens einer Gehäusewand 2 umgebenen Gehäuseinneren und eine im Gehäuse angeordnete temperierbare Inkubatorkammer 3 (auch "Kammer 3") mit einem von mindestens einer Kammerwand umgebenen Kammerinneren zur Aufnahme der Laborproben. Die Außenwände des Gehäuses sind so miteinander verbunden, dass sie alle weiteren Bestandteile des Inkubators tragen, insbesondere auch die Sensoreinrichtung 30. Das Gehäuse ruht auf Sockeln 8. Im bestimmungsgemäßen Gebrauch sind die Außenseiten der Seitenwände 2c des Gehäuses, die Frontwand 2a, die Rückwand 2b sowie die Außenseite der Gehäusetüre 4 und deren Innenseite 4a, sowie die Seitenwände der Kammer, der Kammerfrontwand 3a, und die Kammerrückwand 3b vertikal, also parallel zur Gravitationsrichtung angeordnet. Die obere Außenseite 2d und die nicht sichtbare Bodenseite des Gehäuses und die Bodenwand und Deckenwand der Kammer sind entsprechend horizontal angeordnet. Als Richtung "nach unten" ist im Kontext der Beschreibung der Erfindung immer die Richtung der Gravitation gemeint, anhand der eine bestimmungsgemäß betriebener Inkubator ausgerichtet ist, die Richtung "nach oben" ist die entgegengesetzte Richtung. Die Richtung "nach vorne" bezeichnet die horizontale Richtung zur Vorderseite der geschlossenen Gehäusetüre, die Richtung "nach hinten" bezeichnet die horizontale Richtung zur Rückseite des Inkubators. Die Kammer ist aus Edelstahl gefertigt, das Gehäuse aus lackiertem Metallblech.

Die Gehäusetüre 4 trägt eine Benutzerschnittstelleneinrichtung 5, die hier ein berührungsempfindliches Display umfasst, das vom Benutzer zum Lesen und Eingeben von Informationen verwendet wird, insbesondere zur Ausgabe von mittels der Sensoreinrichtung 20 gewonnen Informationen. Die Gehäusetüre weist zwei Scharniere 9 auf, die die Gehäusetüre mit dem Gehäuse 2 verbinden. Mittels einer Verriegelungseinrichtung 7; 7a, 7b wird die Gehäusetüre in der geschlossenen Position gehalten. Die Gehäusetüre weist einen Türgriff 6 auf.

In Fig. 1c ist die Gehäusetüre 4 geöffnet gezeigt. Die Kammertüre 10 ist mittels der Scharniere 15 an der Kammerfrontwand 3a befestigt, und wird in der gezeigten Position von einem Handverschluss 13 geschlossen gehalten, so dass das Kammerinnere nicht zugänglich ist. Aufgrund der Transparenz der Kammertüre 10 ist das Innere für den Benutzer in dieser Position aber sichtbar. Die Kammertüre wird durch eine umlaufende elastische Dichtung 11 der Kammertüre gasdicht an der Kammerfrontwand gehalten. Die Innenseite 4a der Gehäusetüre weist eine umlaufende elastische Dichtung 14 auf, die im geschlossenen Zustand der Gehäusetüre an der Gehäusefrontwand und der dort umlaufenden Dichtung 12 bündig anliegt und eine gasdichte Abschirmung des Bereichs zwischen Kammertüre 10 und Gehäusetüre 4a erzielt.

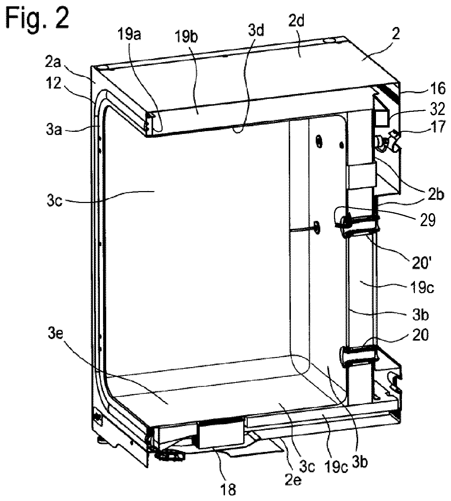

Wie in Fig. 2 teilweise sichtbar ist, weist der Inkubator zwei Temperiereinrichtungen auf, die den Kammerinnenraum 3 temperieren, d.h. deren Temperatur durch eine Temperaturregelung einstellen. Ein Teil der dazu notwendigen Bauteile 18 sind zwischen der Gehäusebodenwand 2e und der Kammerbodenwand 3e angeordnet. Die Heizwendel eines oberen Heizkreises (nicht gezeigt) sind mit der Außenseite der Kammerdeckenwand 3d und einem oberen Bereich der Kammerseitenwände, hier etwa dem oberen 2/3 entlang der Höhe der Seitenwände 3c der Kammer, thermisch gekoppelt und verbunden. Die Heizwendel eines unteren Heizkreises (nicht gezeigt) sind mit der Außenseite der Kammerbodenwand 3e und einem unteren Bereich der Kammerseitenwände, hier etwa dem unteren 1/3 entlang der Höhe der Seitenwände 3c der Kammer, thermisch gekoppelt und verbunden.

Eine thermische Isoliereinrichtung 19 (19a, 19b, 19c) ist zwischen Kammer und Gehäuse vorgesehen. Sie isoliert die Kammer, mit daran anliegenden Temperiereinrichtungen von dem Gehäuse, das an seiner Außenseite direkt mit der Umgebung in Verbindung steht. Der Inkubator arbeitet normalerweise bei Außentemperaturen zwischen 18 °C und 28 °C. Die Temperiereinrichtungen bzw. die Temperaturregelung arbeiten in diesem Bereich besonders effizient. Die Isoliereinrichtung umfasst ein U-förmig gebogenes Isolierelement 19b aus Glaswolle bzw. Mineralwolle, das die Kammerdeckenplatte und die beiden Kammerseitenwände 3c umgibt. Er mündet zum Boden und an die Rückwand hin an Isolierplatten 19c aus PIR-Schaum (Polyisocyanurat-Schaum), und wird zur Frontseite von Gehäuse und Kammer durch einen umlaufenden Nadelvliesstreifen 19a abgeschlossen, der an der Innenseite der Gehäusefrontwand 2a, der Kammerfrontwand 3a und der Dichtung 12 anliegt. Die thermische Isolierung der Kammer nach außen wird durch die erfindungsgemäßen Maßnahmen optimiert.

An der Gehäuserückwand 2b ist eine doppelte Gehäuserückwand 16 zur Abdeckung rückseitig angebrachter Komponenten, insbesondere der Messkammer 31 einer Sensoreinrichtung 30 befestigt. Die Rückwand ist mittels eines Griffs 17 abnehmbar.

Der Inkubator weist an seiner Rückseite zwei Zugangsports 20, 20' auf, die es ermöglichen, durch Öffnungen 20h, 20'h in der Rückwand der Kammer Leitungen, insbesondere mindestens eine Gasleitung zwischen Messkammer 32 und Inkubatorkammer 3, und/oder Kabel ins Innere der Kammer 3 zu verlegen, beispielsweise um eine optional im Inneren angeordnete Sensoreinrichtung anzusteuern. Wenn ein Zugangsport nicht benötigt wird, ist er von einem Stopfen 25 aus thermisch isolierendem Material, z.B. Silikonschaum, gefüllt.

Vorzugsweise mündet eine Gasleitung 29 im Innenraum der Inkubatorkammer 3, führt durch die Öffnung 20h der Kammerrückwand und/oder eine Öffnung im Port 20, zwischen Isoliermaterial 19c der thermischen Isoliereinrichtung 19 entlang der Kammerrückwand, um von dieser mittelbar temperierten Kammerwand temperiert zu werden, und tritt erst dann weg von der Kammerrückwand, durch das Isoliermaterial 19c hindurch in die vorzugsweise vorgesehene Messkammer 32, die hier in dem von der Gehäuserückwand 16 abgetrennten Bereich des Inkubators 1 angeordnet und mit diesem verbunden ist. Eine Abluftleitung der Messkammer 32 (nicht sichtbar) führt vorzugsweise durch die Gehäuserückwand 16 in die Umgebung des Inkubators.

Bevorzugte andere Ausgestaltungen einer Sensoreinrichtung, von mindestens einem VOC-Sensor, und einer Messkammer und deren bevorzugten Anordnungen am Inkubator, insbesondere am Inkubator 1, werden anhand der nachfolgenden Figuren beschrieben.

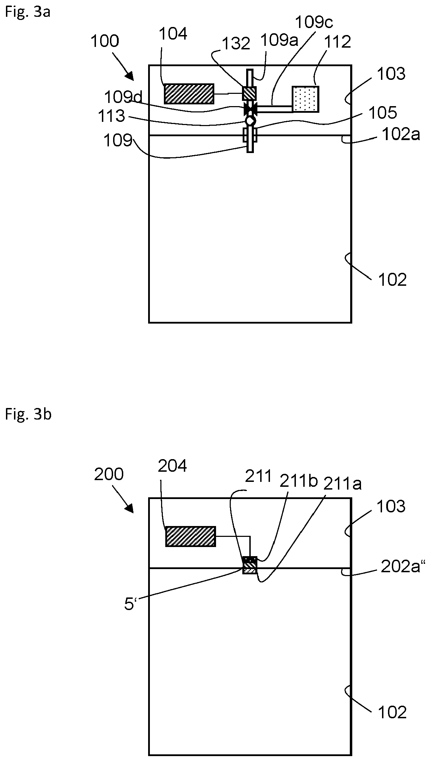

Fig. 3a zeigt: Einen Inkubator 200, mit einer Inkubatorkammer 102 und einem außerhalb der Inkubatorkammer gelegenen Gehäuseabschnitt 103 ("Außenbereich") des Inkubators; diese Bestandteile weist jeder Inkubatoren in den Figuren 3a bis 3k auf. In Fig. 3a wird per Förderung der Kammeratmosphäre mittels Fördermittel 113 (Pumpe oder Ventilator) der Gasaustausch aus dem Inneren der Kammer 102 mittels Gasleitung 109 via Port 105 durch die Kammerwand 102a zu einer Messkammer 132 der VOC-Sensoreinrichtung 130 bewirkt, die mit Steuerung 104 im Außenbereich 103 lokalisiert ist, und Abgase der VOC-Messkammer 132 werden über die Abluftleitung 109a an die Umgebung abgegeben; ein Spülgas 112 wird über eine Spülgasleitung 109c und das Ventil 109d zur VOC-Messkammer geleitet, um diese vor einer Messung zu spülen.

Fig. 3b zeigt: einen Inkubator 200: Eine VOC-Sensor-Steuerung (204) ist im Außenbereich 103 lokalisiert, der Messbereich, nämlich die MOX-Seite 211a des VOC-Sensors 211 liegt im Innenbereich der Kammer 102, eine Heizseite 211b des VOC-Sensors 211 liegt im Außenbereich 103, der VOC-Sensor 211 ist dichtend in Loch 205' in Kammerwand 202a" eingebaut.

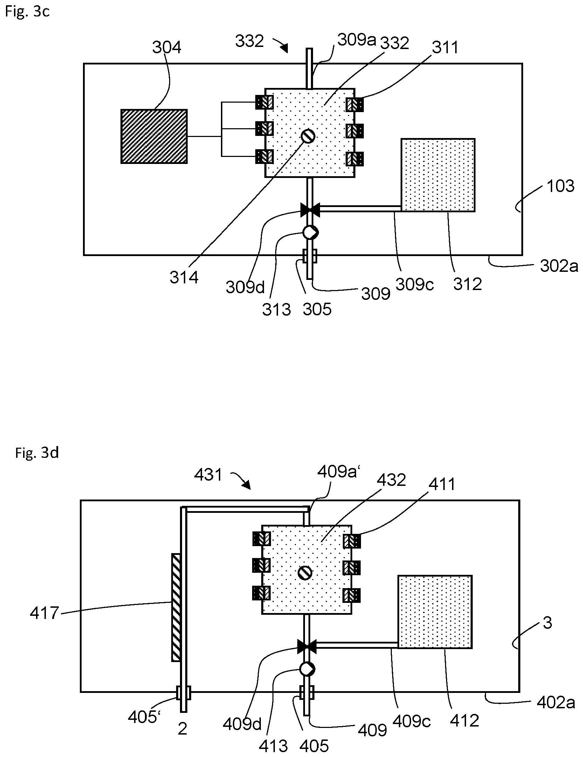

Fig. 3c zeigt: einen Inkubator 300: Per Pumpen 313-Förderung erfolgt der Gasaustausch aus dem Inneren der Kammer 102 mittels Gasleitung 309 via dem Port 305 durch die Kammerwand 302a zu einer Messkammer 332 der VOC-Sensoreinrichtung bzw. e-Nase 331 mit ihren 6 VOC-Sensoren 311, die mit Steuerung 304 im Außenbereich 103 lokalisiert ist, und Abgase von VOC-Messkammer 332 werden über die Abluftleitung 309a an die Umgebung abgegeben; Spülgas 312 wird vor einer Messung über die Spülgasleitung 309c und Ventil 309d zur VOC-Messkammer gefördert.

Fig. 3d zeigt: einen Inkubator 400: Per Pumpen 413-Förderung erfolgt der Gasaustausch aus dem Inneren der Kammer 102 mittels Gasleitung 409 via dem Port 405 durch die Kammerwand 402a zu einer Messkammer 43 der VOC-Sensoreinrichtung bzw. e-Nase 431 mit ihren 6 VOC-Sensoren 411, die mit Steuerung 404 im Außenbereich 103 lokalisiert ist, und Abgase von der VOC-Messkammer 432 werden über die Abluftleitung 409a' und optional den Filter 415 via dem Port 405' zurück durch die Kammerwand 402a in die Kammer 102 geführt; Spülgas 412 wird über die Spülgasleitung 409c und das Ventil 409d zur VOC-Messkammer befördert. Optional ist eine Beheizung bzw. Heizungen 417 der Leitungen 409 vorgesehen, um Kondensation zu verhindern.

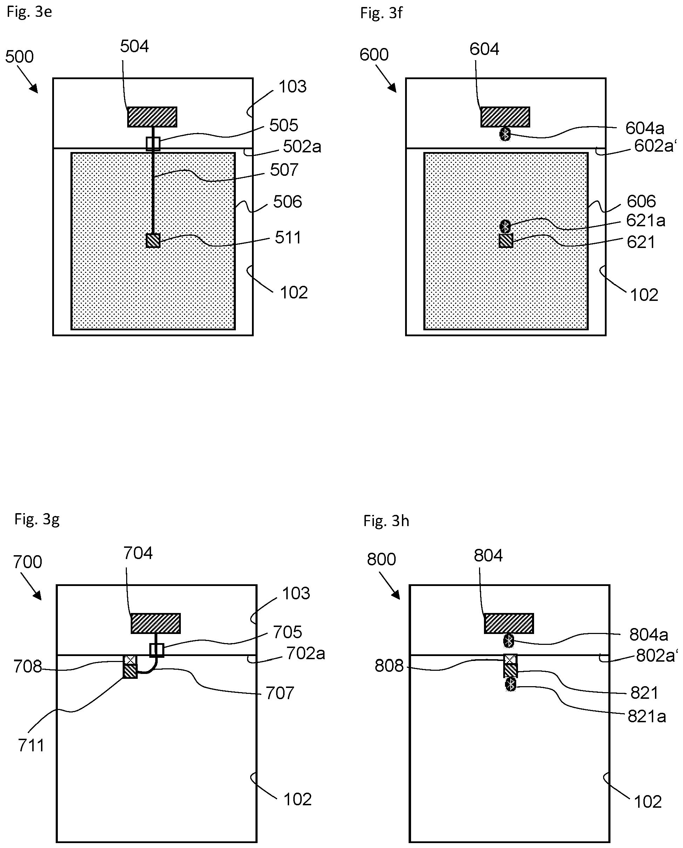

Fig. 3e zeigt: einen Inkubator 500: ein VOC-Sensor 511 ist auf einem Regalbrett 506 in der Kammer 102 angeordnet und mit dem Kabel 507 und kabel-basierter Signalverbindung via dem Port 505 durch eine Kammerwand 502a zu einer VOC-Steuerung 504 im Außenbereich 103 verbunden.

Fig. 3f zeigt: einen Inkubator 600: ein VOC-Sensor 621 ist auf einem Regalbrett 606 in der Kammer 10 montiert und mit kabelloser, funk 621a, 604a-basierter Signalverbindung durch eine portfreie Kammerwand 602a' mit einer VOC-Steuerung 604 im Außenbereich 103 verbunden.

Fig. 3g zeigt: einen Inkubator 700: ein VOC-Sensor 711 ist mittels Magnethefter 708 in der Kammer 102 an der Kammerwand 702a befestigt und mit einer Kabel 707-basierter Signalverbindung via dem Port 705 durch die Kammerwand 702a mit einer VOC-Steuerung 704 im Außenbereich 103 verbunden.

Fig. 3h zeigt: einen Inkubator 800: ein VOC-Sensor 821 ist mittels Magnethefter 808 in der Kammer 102 mit der Kammerwand 802a' verbunden mit kabelloser, funk 821a, 804a-basierter Signalverbindung durch eine portfreie Kammerwand 802a' mit einer VOC-Steuerung 804 im Außenbereich 103 verbunden.

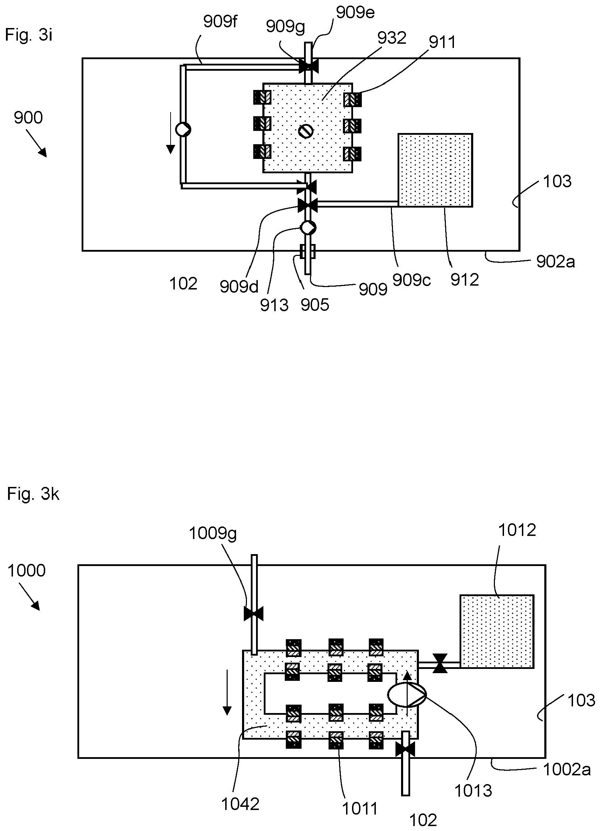

Fig. 3i zeigt: einen Inkubator 900: gezeigt ist eine Messkammer mit Ringverlauf und zirkulierendem Kammergas: Per Pumpen 913-Förderung erfolgt der Gasaustausch aus dem Inneren der Kammer 102 mittels der Gasleitung 909 via dem Port 905 durch die Kammerwand 902 zu einer Messkammer 932 der VOC-Sensoreinrichtung bzw. e-Nase 931 mit ihren hier 6 VOC-Sensoren 911d, die mit der Steuerung 904 im Außenbereich 103 lokalisiert ist, und nach Schließen der Ventile 909d und 909g werden Abgase von der VOC-Messkammer 932 über Zirkulierleitungen 909f mittels Pumpe 916 zirkulierend durch die Messkammer 932 gefördert, um eine kontinuierliche Konvektion des VOC-enthaltenden Kammergases ohne größeren Verlust von Kammer 102-Gas bzw. Atmosphäre zu ermöglichen; Spülgas 912 wird über eine Spülgasleitung 909c und ein Ventil 909d zur VOC-Messkammer befördert. Infolge des zirkulierenden Gasvolumens wird über dieses hinaus kein Gasvolumen der Kammer 102 entnommen, wodurch der Kammergasverlust minimiert ist.

Fig. 3k zeigt: einen Inkubator 1000: gezeigt ist eine zirkular gestaltete Messkammer für Messung an zirkulierendem Gas: Per Pumpen 1013-Förderung erfolgt der Gasaustausch aus dem Inneren der Kammer 102 mittels der Gasleitung 1009d via dem Port 1005 durch die Kammerwand 1002a zu einer ringförmigen Messkammer 1042 der VOC-Sensoreinrichtung bzw. e-Nase 1041 mit ihren hier 12 VOC-Sensoren 1011, und nach Schließen der entsprechenden Ventile werden Abgase zirkulierend durch die VOC-Messkammer 1042 mittels der Pumpe 1013 gefördert, um kontinuierliche Konvektion des VOC-enthaltenden Kammergases ohne größeren Verlust von Kammer 102-Gas bzw. Atmosphäre zu ermöglichen; ein Spülgas 1012 wird über die Spülgasleitung 1009c und das Ventil 1009d zur VOC-Messkammer befördert. Vorteile: das Kammergas wird auf kürzestem Weg der Kammer entnommen, es erfolgt somit kaum Kondensation; entnommen werden muss der Kammer 102 aber nur das für die schmale ringförmige VOC-Messkammer benötigte Volumen; und während der Messung zirkuliert das Gasvolumen; in Folge geht im Vergleich zum Aufbau mit Gaszuleitung und Abgasleitung zur Umgebung weniger Kammergas verloren.

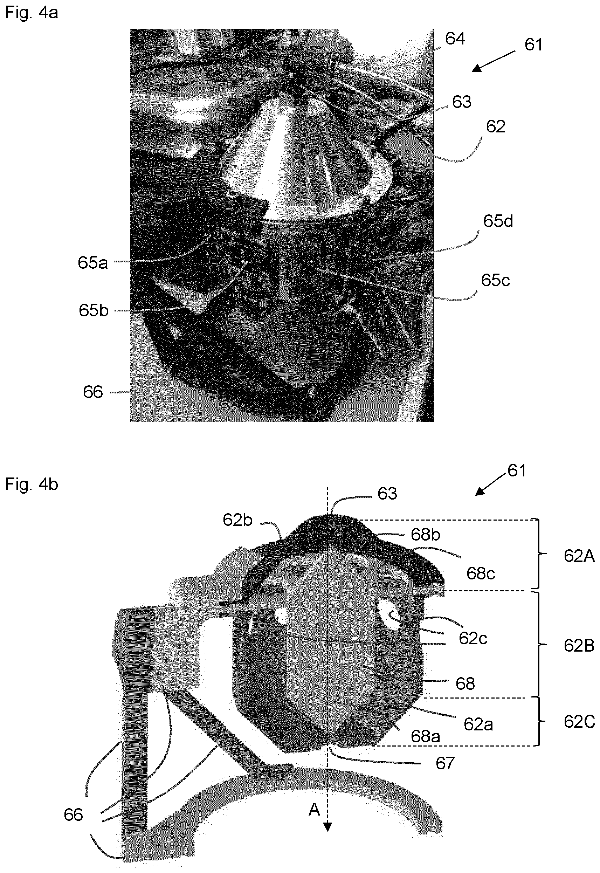

Fig. 4a zeigt eine Sensoreinrichtung 61 mit Messkammer 62 gemäß einem Ausführungsbeispiel der Erfindung in einer perspektivischen Seitenansicht von schräg oben, die insbesondere im Inkubator 1 der Figuren 1a bis 2 verwendet werden kann. Die Messkammer ist ein hohlspindelförmiger Körper, der in der Fig. 4a an seinem oberen Ende eine Zuflussöffnung 63 für den Zufluss von Atmosphärengas aus der Inkubatorkammer mittels der Gasleitung 64 erlaubt, und einen Abfluss durch die Abflussleitung 67. Die Messkammer ist mittels einer Halterung 66 am Inkubator befestigbar, insbesondere verschraubbar, verlötbar oder anders unbeweglich am Inkubator, insbesondere einer Gehäusewand oder Kammerwand verbindbar. Die Messkammer 62 weist einen Teilhohlkörper 62a auf, in den ein Gasführungskörper 68 gesetzt wird, und der mit einem Deckelteil 62b abgedeckt wird. Die Teile 62a, 6sb und 68 sind fest miteinander verbunden. An der Außenseite der Messkammer 62 sieht man die Rückseiten 65a bis 65i der -hier neun- MOX-VOC-Sensoren, die jeweils einen Messbereich bzw. eine beheizte MOX-Adsorptionsfläche (nicht sichtbar) an ihrer Vorderseite aufweisen, die der zentralen Längsachse A der Messkammer zugewandt und parallel zu dieser angeordnet sind.

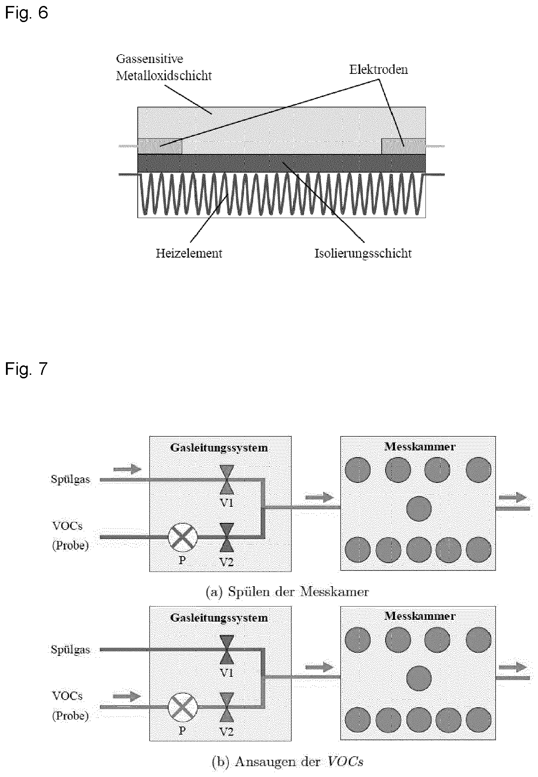

Fig. 4b zeigt eine durch Schnitt des Modells geöffnete Ansicht der Sensoreinrichtung 61 mit Messkammer der Fig. 6a in einer perspektivischen Seitenansicht, ohne eingesetzte VOC-Sensoren und ohne deren Kabel.

Die Messkammer 62 weist, wie in Fig. 4b gezeigt ist einen oberen hohlkonusförmigen Abschnitt 62A auf, einen mittleren hohlzylinderförmigen Abschnitt 62B und einen unteren hohlkonusförmigen Abschnitt 62C. An der Spitze des hohlkonusförmigen Abschnitt 62A ist die Zuflussöffnung 63 für das Gas vorgesehen, an der Spitze des hohlkonusförmigen Abschnitt 62C ist die Abflussöffnung 63 für das Gas vorgesehen. Die Hauptströmungsrichtung A ergibt sich aus der geraden Verbindung der Zentren der kreisförmigen Zuflussöffnung 63 und der kreisförmigen Abflussöffnung 67. An der der Zuflussöffnung zugewandten Spitze des Abschnitts 68b des Gasführungskörpers ist ein Umgebungssensor (nicht sichtbar; Druck, Temperatur, Luftfeuchte) angeordnet, der -ebenso wie die neun VOC-Sensoren, mit der elektronischen Auswertungseinrichtung für den Signalaustausch verbunden sind.

Der Gasführungskörper ist spindelförmig und so koaxial zur Achse A mit dem hohlspindelförmigen Verlauf der Außenwand der Messkammer 62 angeordnet, dass sich zwischen der Innenseite der Außenwand der Messkammer 62 und der Außenseite des Gasführungskörpers ein hülsenförmiger, bzw. ein Strömungskanal mit ringförmigen Querschnitten ergibt. Das Gas wird auf diese Weise gleichförmig und insbesondere unter Eliminierung von Wirbelbildung -also möglichst laminar- an den Messbereichen der VOC-Sensoren 65a-65i vorbeigeführt, die an den kreisförmigen Öffnungen 62c der Außenwand der Messkammer 62 dicht anliegen, so dass an jeder der neun Öffnungen 62c dieselbe Fläche einer MOX-Adsorptionsfläche des Messbereichs des jeweiligen VOC-Sensors im Kontakt mit dem parallel zur Strömungsrichtung A vorbeiströmendem Gas steht. Durch die Führungselemente und die Gleichförmigkeit des Gasstroms wird die mittels der Sensoreinrichtung 61 durchgeführte Messung besonders sensibel, zudem reproduzierbar und zuverlässig. Indem die Rückseite der MOX-VOC-Sensoren außerhalb der Messkammer und nicht im Kontakt mit dieser angeordnet sind, wird der Wärmeübertrag zwischen den Heizelementen der Sensoren und der Messkammer minimiert, die Rückseiten sind zudem einfach per Konvektion/Luftstrom kühlbar.

Ausführungsbeispiel eines Inkubators mit Sensoreinrichtung und elektronischer Nase

Der nachfolgend beschriebene erfindungsgemäße Inkubator weist den Aufbau gemäß den Figuren 1a bis 2 auf und verwendet eine Sensoreinrichtung 61 aus Fig. 4a, 4b (zur Realisierung der Sensoreinrichtung 30). Die Sensoreinrichtung 61 ist als elektronische Nase mit insgesamt neun verschiedenen VOC-Sensoren ausgebildet.

Grundlegendes zu VOCs:
Beim Stoffwechsel von mikrobiellen Organismen und Zellen VOCs freigesetzt. Die Sensoreinrichtung 61 ist nach dem Prinzip einer Elektronische Nase aufgebaut und misst VOCs. Sie ermöglicht einen Rückschluss auf die Kontamination einer Zellkultur bzw. auf Vorgänge in Zellkulturen oder in der Inkubatorkammer, die mit Änderungen der VOC-Konzentration in der Inkubatorkammer verbunden sind. Voraussetzung hierfür ist, dass die Gassensoren selektiv und sensitiv genug für die auftretenden VOCs sind, um den Nachweis der mikrobiellen Kontamination zu führen.

Es werden hier insgesamt 9 Gassensoren mit möglichst unterschiedlicher Selektivität verbaut. Diese reagieren somit unterschiedlich auf die VOCs eines mikrobiellen Organismus und erzeugen damit ein charakteristisches Messsignalmuster. Die Messung findet insbesondere im Zeitraum des biologischen Probenwachstums statt, weshalb das Messsignalmuster zeitabhängig erfasst werden kann. Das Messsignalmuster der biologischen Probe enthält Informationen, die analysiert und in eine semantische Aussage gebracht werden sollen. Näheres zur Nachweisbarkeit von mikrobieller Kontamination einer Zellkultur wird nachfolgend noch beschrieben.

Durch die Vielfalt der Gassensoren kann eine bessere Unterscheidung zwischen verschiedenen mikrobiellen Organismen und Zellen getroffen werden. Bspw. entsteht teilweise bei verschiedenen Kontaminanten ein ähnliches Messsignalmuster, aber der Signalverlauf einzelner Gassensoren unterscheidet sich charakteristisch. Der Vorteil der Verwendung mehrerer verschiedener Gassensoren ist also die Erhöhung des Informationsgehalts der Messung.

Die Sensoreinrichtung 61 verfügt über eine Messkammer (MK), ein Gasleitungssystem (GS) und eine Verarbeitende Einheit (VE). Die MK beinhaltet die Gassensoren (VOC-Sensoren), welche die VOCs messen. Das GS leitet die VOCs mithilfe von Aktuatoren zur MK. Die VE steuert das GS, liest die Gassensoren und den Umgebungssensor aus, verarbeitet die Daten und stellt eine Kommunikationsschnittstelle zum Inkubator bereit. Die VE beinhaltet eine elektronische Steuerungseinrichtung der Sensoreinrichtung, welche die eine Datenverarbeitungseinrichtung aufweisende Auswertungseinrichtung beinhaltet. Ein Mikrocontroller der Steuerungseinrichtung ist hier der Raspberry Pi 3 B (Raspberry). Die Kommunikationsschnittstelle ermöglicht den Informationsfluss zwischen einem AI-Modul und Messkammer und somit die Steuerung der Sensoreinrichtung 61 mithilfe des Nutzerinterfaces.

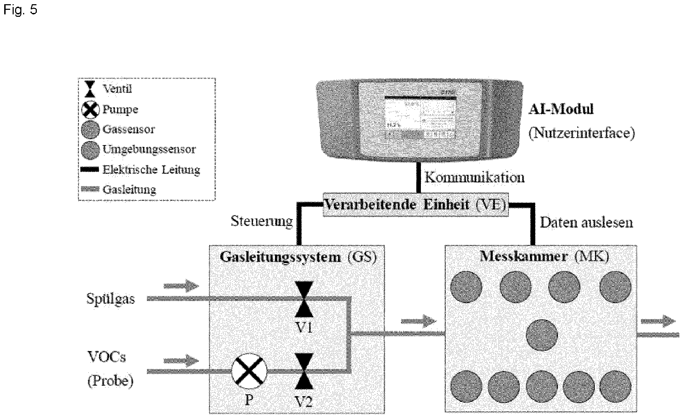

In Fig 5 ist gezeigt: der schematische Aufbau der Sensoreinrichtung 61 und deren Verbindung zu einer Steuereinrichtung des Inkubators, auch bezeichnet als AI-Modul. Die MK beinhaltet die Gassensoren, den Umgebungssensor, einen Ein- und einen Ausgang. Das GS leitet mithilfe von Gasleitungen der Ventile und der Pumpe entweder Spülgas oder VOCs (also Kammeratmosphärengas) in die MK. Die VE ist über elektrische Leitungen mit dem GS und der MK verbunden. Sie steuert das GS, ließt die Gassensoren und den Umgebungssensor aus, verarbeitet die Daten und stellt eine Kommunikationsschnittstelle zum Inkubator bereit. Die Kommunikationsschnittstelle ermöglicht den Informationsfluss zwischen AI-Modul und Sensoreinrichtung 61 und somit die Steuerung der Sensoreinrichtung 61 mithilfe des Nutzerinterfaces (Benutzerschnittstelleneinrichtung).

Damit vor jeder VOC-Analyse in diesem Ausführungsbeispiel möglichst immer dieselben Startbedingungen geschaffen werden, wird vor dem Einleiten der VOCs die MK gespült. Der Spülvorgang ist vorteilhaft für das Generieren von vergleichbaren Messergebnissen. Wenn die Sensoreinrichtung 61 nicht benutzt wird, können entweder Gase aus der Umgebung in die MK eindringen oder VOCs der vergangenen VOC-Messung in der MK zurückbleiben. Vorzugsweise wird immer ein Spülgas konstanter Zusammensetzung verwendet. Um den Anteil von sich ändernden Gasen im Spülgas gering zu halten, wird vorzugsweise Stickstoff 5.0 als Spülgas verwendet. Dieser besitzt hier einen Reinheitsgrad von >99.999%.

Die MK hat einen Ein- und Ausgang (Zu- und Abfluss). Über den Eingang werden Spülgas oder VOCs in die MK eingeleitet. Vor dem Eingang befindet sich eine Y-Kopplung, welche die Spül- und VOC-Leitung zusammenführt. Über den Ausgang entweichen die Gase wieder aus der MK. Der Ein- und Ausgang befinden sich vorzugsweise gegenüber und mittig auf einer jeweiligen Außenwand der MK, um einen möglichst gleichmäßigen Gasdurchfluss und -verteilung zu gewährleisten.

Als Gassensoren (VOC-Sensoren) werden vorzugsweise MOX-Sensoren verwendet.

Es existieren verschiedene Analysemethoden, um VOCs zu detektieren, wozu u.A. auch die Elektronische Nase gehört. Eine Elektronische Nase nutzt ein Sensor-Array, um mithilfe von Mustererkennung einen Fingerabdruck für einen gegebenen Geruch zu generieren und diesen von Fingerabdrücken anderer Gerüche zu unterscheiden. Auf diese Weise mimt eine Elektronische Nase das olfaktorische System von Säugetieren nach und erlaubt das Erkennen von Gerüchen als Ganzes und damit die Quelle des Geruchs ausgemacht werden kann. So kann bspw. die Identifikation von Mikroorganismen erfolgen, indem aufgrund der detektierten Mixtur von charakteristischen VOCs ein Rückschluss auf die Quelle gezogen wir.

Das Messsystem einer elektronische Nase ist insbesondere aufgebaut aus einer Probenleitenden Einheit, Detektionseinheit als auch Berechnungseinheit und die verwendeten Gassensoren werden vorzugsweise so gewählt, dass diese sensitiv für die auftretenden Gasmoleküle sind, aber die einzelnen Gassensoren unterschiedlich stark auf diese reagieren. Hier werden Metalloxid-Halbleiter (MOX) Gassensoren verwendet, welche zu der Klasse der chemischen Sensoren gehören. Chemische Sensoren besitzen eine Detektionsschicht, mithilfe einer chemischen Interaktion in ein elektrisches Signal transformiert werden kann und sind darüber hinaus nicht nur kostengünstig, sondern können auch im kontinuierlichen Messbetrieb verwendbar.

Der Aufbau und die Funktionsweise von MOX-Sensoren: Der Sensormechanismus basiert darauf, dass abhängig von der Konzentration des Zielgases die elektrische Leitfähigkeit der gassensitiven Metalloxidschicht bzw. des Halbleiters verändert und damit die Anwesenheit als auch Menge des Zielgases bestimmt wird. Typischerweise besteht ein MOX-Sensor aus vier Elementen: Gassensitive Metalloxidschicht, Elektroden, Heizelement und Isolierungsschicht (siehe Fig. 6).

Fig. 6 zeigt den schematischer Aufbau eines MOX-Sensors. Ein MOX-Sensor besteht aus 4 Elementen: Gassensitive Metalloxidschicht, Kontaktelektroden, Heizelement und Isolierungsschicht. Das Heizelement ist von der gassensitiven Metalloxidschicht und den Kontaktelektroden durch die Isolierungsschicht getrennt. Die gassensitive Metalloxidschicht wird durch das Heizelement erhitzt und Sauerstoffmoleküle aus der Umgebung werden an der Oberfläche der gassensitiven Metalloxidschicht adsorbiert. Die adsorbierten Sauerstoffmoleküle fangen Elektronen von den leitenden Bändern des Halbleiters ein und es bilden sich energetische Barrieren, die so einen Teil des Elektronenflusses im Halbleiter blockieren und somit die elektrische Leitfähigkeit verschlechtern bzw. den Widerstand des Gassensors erhöhen. Sobald reduzierende Gase (Zielgase) präsent sind, reagieren diese mit den gebundenen Sauerstoffmolekülen. Die Sauerstoffmoleküle werden von der Oberfläche der gassensitiven Metalloxidschicht gelöst und die Leitfähigkeit steigt bzw. der Widerstand sinkt.

Hersteller geben in der Regel Umgebungsbedingungen an, in denen die MOX-Sensoren valide Messwerte liefern und wie stark sie von diesen beeinflusst werden. Um zu überblicken, wie sehr die Umweltbedingungen einen Einfluss auf die VOC-Messungen haben, wird zusätzlich ein Umgebungssensor mittig in der MK angebracht. Zu den relevanten Umweltbedingungen zählen die Feuchtigkeit, Temperatur und der Umgebungsdruck.

Der Prozess einer VOC-Messung ist vorzugsweise in zwei Phasen eingeteilt - das Spülen und Einleiten der VOCs. Der Inkubator mit Sensoreinrichtung wird zunächst für die Messung initialisiert, es wird gespült. Die Gassensoren werden für die Messung vorzugsweise kontinuierlich ausgelesen und zwischengespeichert. Bei Bedarf kann der Nutzer die zwischengespeicherten Daten dauerhaft abspeichern und ggf. exportieren. Die VOC-Messung selbst wird vom Nutzer oder automatisch vom Inkubator durch das Steuern des GS gestartet oder beendet. Die zu steuernden Elemente sind die Ventile und die Pumpe. Zum Starten der VOC-Messung wird im Beispiel der Spülvorgang eingeleitet. Ventil 1 (V1) ist geöffnet, Ventil 2 (V2) geschlossen und die Pumpe (P) deaktiviert (siehe Fig. 7 oben). Wenn der Spülvorgang beendet und die Zufuhr der VOCs gestartet werden soll, wird Ventil 1 (V1) geschlossen, Ventil 2 (V2) geöffnet und die Pumpe (P) gestartet (siehe Fig. 7 unten). Zum Beenden der VOC-Messung wird die Pumpe (P) ausgeschaltet und Ventil 2 (V2) geschlossen. Die Sensoreinrichtung wird vorzugsweise über ein Netzteil des Inkubators mit Spannung versorgt. Dies betrifft die VE, die Gassensoren der MK, die Ventile und die Pumpe des GS.

Fig. 7 zeigt schematisch die Gassteuerung bei einer VOC-Messung. Diese ist vorzugsweise ist in Spülen und Ansaugen von VOCs aufgeteilt. Gas fördernde Bauteile werden grün markiert und nicht fördernde rot. Während des Spülvorgangs ist Ventil 1 geöffnet, Ventil 2 geschlossen, die Pumpe deaktiviert und das Spülgas fließt durch die Messkammer. Während des Ansaugens der VOCs ist Ventil 1 geschlossen, Ventil 2 geöffnet, die Pumpe aktiviert und die VOCs fließen durch die Messkammer.

Die MK besteht aus einer Aluminium-Spritzgusskammer mit anschraubbaren Deckel und beinhaltet die typenunterschiedlichen Gassensoren (MQ 1, MQ 2, MQ 3, MQ 4, MQ 5,MQ 6, MQ7,MQ 8, MQ9 und MQ135 bzw. Bezugszeichen 65a-i; herkömmlich bezogen von der HANWEI ELETRONICS CO.,LTD) und den Umgebungssensor (BME680). Der Umgebungssensor stellt die geforderten Umweltparameter Temperatur, Feuchtigkeit und Druck bereit und wurde innerhalb der MK platziert. Die Gassensoren wurden so gewählt, dass sie für die potentiell auftretenden Stoffgruppen der VOCs größtenteils selektiv sind und wurden gemäß des aufgestellten Konzepts anliegend an die MK platziert. Die Anschlussstellen zwischen Gassensoren und MK wurden mit Silikon abgedichtet. Die jeweilige Selektivität der Gassensoren kann in Tabelle 1 unter der Details-Spalte eingesehen werden.

**Tabelle 1 Hardwareteile der Messkammer (MK)**

| Messkammer (*MK*) | | |
|---|---|---|
| Bauteil | Bezeichnung | Details |
| Gassensor | MQ 2 | Alkane (Butan, Propan, Methan) |
| | | Alkohole |
| | | Wasserstoff |
| Gassensor | MQ 3 | Alkohole |
| Gassensor | MQ 4 | Alkane (Methan) |
| Gassensor | MQ 5 | Alkane (Methan, Propan, Butan) |
| Gassensor | MQ 6 | Alkane (Methan, Propan, Butan) |
| Gassensor | MQ 7 | Oxide (Kohlenstoffmonoxid) |
| Gassensor | MQ 8 | Wasserstoff |
| Gassensor | MQ 9 | Kohlenstoffmonoxid Alkane (Methan, Propan, Butan) |
| Gassensor | MQ 135 | Alkohole |
| | | Benzole (Benzol) |
| | | Amine (Ammoniak) |
| | | Oxide (Kohlenstoff-, Stickstoffdioxid) |
| Umgebungssensor | Adafruit BME680 | Temperatur, Feuchtigkeit, Druck |
| Spritzgusskammer Silikon | Hammond | Aluminium, 170 x 120 x 55 mm Abdichten Gassensoren |

Als Testprobe zum Nachweis der Funktionsweise der Sensoreinrichtung und zur Erzeugung von Kontamination in der Inkubatorkammer wurden Escherichia coli Bakterien des Stamms DH5a (DH5a). Diese sind häufig im Laboralltag anzutreffen. Verschiedene VOCs, werden von den DH5a ausgestoßen, siehe Tabelle 2. Die VOCs gehören zu den Stoffgruppen der Benzole, Alkylbenzole, Ketone, Alkohole, Alkane, Terpene, Säuren, Carbonsäuren, Ester, Aldehyde, Alkene, Heterocyclische Amine und Indolen. Der größte Anteil der aufgeführten VOCs gehört zu der Stoffgruppe der Alkohole. Ein Teil der verwendeten Gassensoren sind nach den Angaben des Herstellers für Gase der Stoffgruppen der Alkohole, Alkane, Benzole und Amine selektiv. Dem entsprechend sollten die Gassensoren MQ2, MQ3, MQ4, MQ5, MQ6, MQ9 und MQ135 auf die VOCs der DH5 ansprechen und ein Anstieg der Messsignale zu verzeichnen sein. Da der größte Anteil der entstehenden VOCs zur Gruppe der Alkohole gehören, erzeugen die Gassensoren MQ2, MQ3 und MQ135 höhere Messsignale als die anderen Gassensoren.

**Tabelle 2 DH5α emittierte VOCs (in IUPAC-Schreibweise) und deren chemische Klassifizierung [4].**

| IUPAC-Name | Chemische Klassifizierung |
|---|---|
| 1,2,3-Trimethylbenzol | Benzole, Alkylbenzole |
| 1-(4-Methylphenyl)ethanon | Benzole, Ketone |
| 1,4-Xylen | Benzole, Alkylbenzole |
| 2-(4-Methyl-3-cyclohexen-1-yl)-2-propanol | Alkohole, Terpene |
| 2-Ethyl-1-hexanol | Alkohole |
| 2-Phenylethanol | Alkohole |
| 3-Hydroxy-2-butanon | Alkohole, Ketone |
| 2,3-Butandiol | Alkohole |
| 2-Decanol | Alkohole |
| Dodecan | Alkane |
| Octadecan | Alkane |
| Hexansäure | Säuren, Carbonsäuren |
| Nonansäure | Säuren, Carbonsäuren |
| Octansäure | Säuren, Carbonsäuren |
| Ethyl-octanoat | Ester |
| 3-Methylbutyl-acetat | Ester |
| Lauraldehyd | Aldehyde |
| 2-Methylpentanal | Aldehyde |
| 4-Methyl-1-hexen | Alkene |
| 1H-Indol | Indole, Heterocyclische Amine |

Chinese Hamster Ovary Zellkulturen (CHO) sind häufig im Laboralltag anzutreffende Zelltypen. Die von CHO abgegebenen VOCs gehören zu den Stoffgruppen der Alkane, Aldehyde, Ester, Benzole, Ketone, Pyrazole, Oxime und Alkohole. Der größte der aufgeführten VOCs gehört zu der Stoffgruppe der Alkane. Ein Teil der verwendeten Gassensoren sind nach den Angaben des Herstellers für Gase der Stoffgruppen der Alkohole, Alkane und Benzole selektiv. Dem entsprechend sollten die Gassensoren MQ2, MQ3, MQ4, MQ5, MQ6, MQ9 und MQ135 auf die VOCs der CHOs ansprechen und ein Anstieg der Messsignale zu verzeichnen sein. Da der größte Anteil der entstehenden VOCs zur Gruppe der Alkane gehören, sollten die Gassensoren MQ2, MQ4, MQ5, MQ6, und MQ9 höhere Messsignale als die anderen Gassensoren erzeugen. Wie auch bei den DH5 müssen die Zellkulturen bei jedem Versuch eine gleichbleibende Wachstumsdynamik aufweisen. Im Gegensatz zu den DH5 wurden die CHOs nicht eigenständig gezüchtet, sondern vom Anmelder bereitgestellt und nach den internen Standardprozeduren gezüchtet.

Die Auswertungseinrichtung (VE) verwendet einen Algorithmus, um zu erkennen, ob in einer Testprobe (CHO mit/ohne DH5a) eine Kontamination vorliegt oder nicht. Der entwickelte Algorithmus basiert auf der sequentiellen CUSUM-Analysetechnik (auch CUSUM Control Chart genannt) und wurde zuerst von Page vorgestellt. Auch ein KI-Algorithmus, insbesondere ein neuronales Netz wäre zur Auswertung möglich. Die CUSUM-Analysetechnik wird genutzt, um die Abweichungen eines laufenden Prozess zu überwachen. xᵢ sei die i-te Beobachtung des Prozesses. Der Prozess wird in zwei Zustände eingeteilt - entweder ist er unter Kontrolle oder nicht. Wenn der Prozess unter Kontrolle ist, unterliegt xᵢ einer Normalverteilung mit einem Mittelwert µ0 und einer Standardabweichung σ. µ0 wird häufig als Zielwert interpretiert, dem xᵢ möglichst nahe sein muss, damit der Prozess unter Kontrolle bleibt.

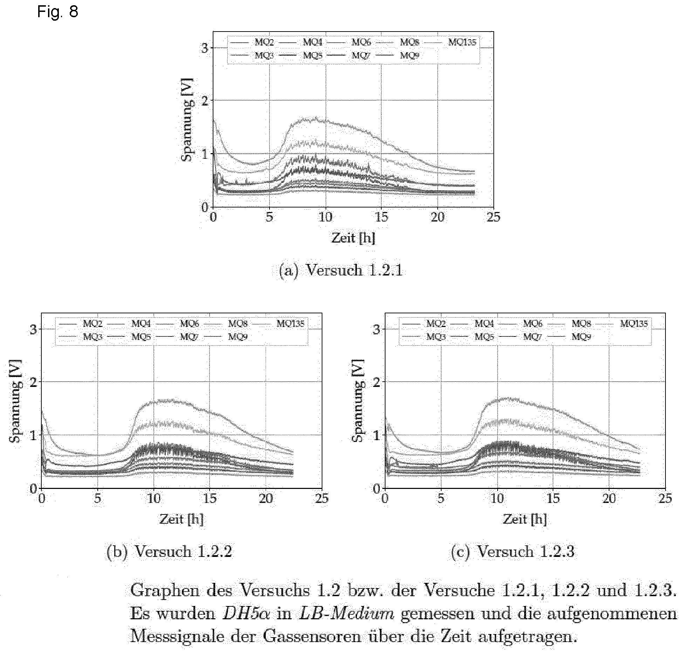

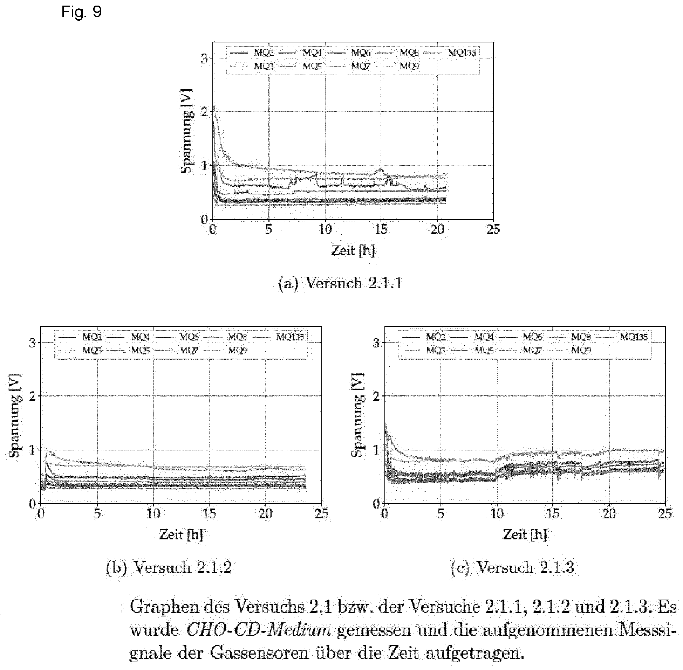

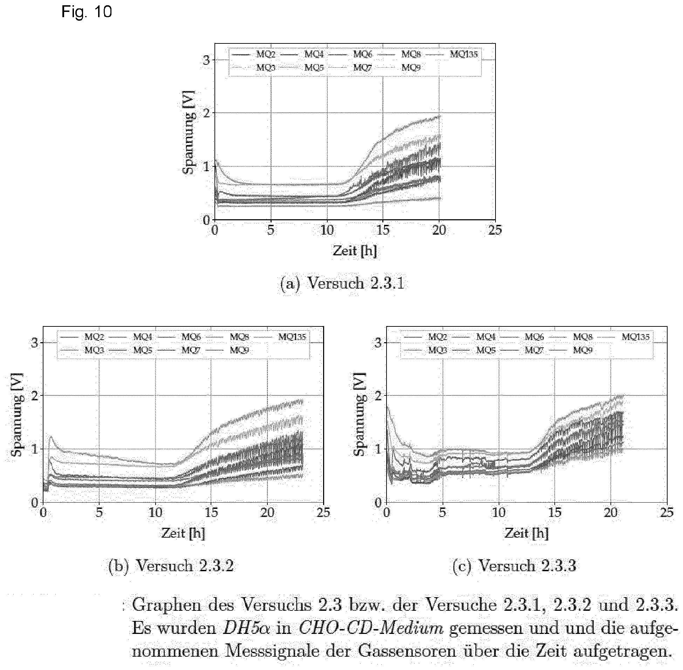

Fig. 8 zeigt Diagramme mit Messwerte zu einer Messung von VOCs aus DH5a, die in Zellkulturflaschen in der Inkubatorkammer des erfindungsgemäßen Inkubators wuchsen. Es wurden DH5a in LB-Medium gemessen und die aufgenommenen Messsignale der Gassensoren über die Zeit aufgetragen. Zur Erfassung der in Fig. 8 (analog: Fig. 9, 10) gezeigten Messdaten führt eine Steuereinrichtung des Inkubators, insbesondere mittels der Programmierung einer Datenverarbeitungseinrichtung des Inkubators, folgende Schritte aus: innerhalb eines ersten Zeitraums ab Start der Messung zum Zeitpunkt Null wird die Messkammer, welche die Messbereiche der typenunterschiedlichen MOX-Sensoren aufweist, mit einem Spülgas, hier Stickstoff, gespült. Dabei messen die MOX-Sensoren (jeweils in Form eines Spannungswertes) innerhalb dieses ersten Zeitraums im Wesentlichen zeitlich parallel und sukzessive Referenzmesswerte, während das Spülgas an den Messbereichen vorbeiströmt. Dieser erste Zeitraum liegt in Fig. 8a bei 5 Stunden, in Fig. 8b und 8c bei 7 Stunden. In einem sich unmittelbar an den ersten Zeitraum anschließenden zweiten Zeitraum wird eine als Zuluftkanal dienende Gasleitung mittels eines Ventils geöffnet, das gleichzeitig den Zustrom von Spülgas in die Messkammer stoppt. Dadurch strömt ein Volumen der Gasatmosphäre in die Messkammer - und aus dieser beispielsweise durch einen Abluftkanal wieder ab. Das Volumen strömt während des zweiten Zeitraums an den Messbereichen vorbei. Dabei messen die MOX-Sensoren innerhalb dieses zweiten Zeitraums im Wesentlichen zeitlich parallel und sukzessive Messwerte. Der zweite Zeitraum liegt in Fig. 8a zwischen dem Ende der 5. Stunde, in Fig. 8b und 8c zwischen dem Ende der 7. Stunde ab Start der Messung bis jeweils zum Ende des Zeitraums 22,5 Stunden ab Start der Messung. Für jeden MOX-Sensor bilden die innerhalb des ersten Zeitarums ermittelten Referenzmesswerte eine "Basislinie", im Vergleich zu der der Messwert betrachtet wird: die Differenz aus Messwert und Referenzmesswert zu einem bestimmten Zeitpunkt kann als Ergebnis der Messung der Kontamination betrachtet werden (Ergebnismessdaten). Ist diese Differenz Null, liegt keine Kontamination vor. Ist sie hier im Beispiel größer Null, liegt eine Kontamination vor. Die meisten MOX-Sensoren detektieren eine Kontamination durch von Null verschiedene Ergebnissmessdaten.

Bei dieser Art Messung (Fig. 8, 9, 10) und nachfolgender Auswertung ist die Datenspeichereinrichtung zur Durchführung der folgenden Schritte programmiert: i) Speichern eines Messdatensatzes im einem Datenspeicher, der hier Messwerte der Anzahl N=9 >1 von VOC-Sensoren beinhaltet, wobei ein Messwert für das detektierte Messignal des jeweiligen VOC-Sensors charakteristisch ist, das in Gegenwart eines aus der Inkubatorkammer stammenden und am Messbereich des VOC-Sensors anliegenden Volumens der Gasatmosphäre erfasst wurde; ii) Ermitteln von ersten Ergebnismessdaten aus einem Vergleich des Messdatensatzes mit einem Referenzdatensatz, insbesondere unter Verwendung einer Differenz des Messdatensatzes und des Referenzdatensatzes, der, insbesondere zeitabhängig erfasste, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung stammenden und am Messbereich des VOC-Sensors anliegenden Spülgases erfasst wurde;
Optional kann auch der Schritt vorgesehen sein: iii) Erkennen eines charakteristischen Datenmusters in dem die Ergebnismessdaten enthaltenden Ergebnismessdatensatz, wobei das charakteristische Datenmuster ein bestimmtes, im Atmosphärengas nachgewiesenes VOC bzw. VOC-Gemisch, insbesondere auch dessen Konzentration oder Menge, repräsentiert. Beispielsweise können die Ergebnismesswerte der typenunterschiedlichen MOX-Sensoren, insbesondere unter Berücksichtigung eines gemeinsamen Skalierungsfaktors, bzw. unter Berücksichtigung eines Normierungsfaktors, das charakteristische Datenmuster zu einem Zeitpunkt oder zu mehreren Zeitpunkten der Messung erfassen.

Fig. 9 zeigt Diagramme mit Messwerte zu einer Messung von VOCs aus CHO-CD-Medium, die in Zellkulturflaschen in der Inkubatorkammer des erfindungsgemäßen Inkubators wuchsen. CHO-CD-Medium wurden mittels der Sensoreinrichtung 61 gemessen und die aufgenommenen Messsignale der Gassensoren über die Zeit aufgetragen.

Fig. 10 zeigt Diagramme mit Messwerten zu einer Messung von VOCs aus DH5a in CHO-CD-Medium, die in Zellkulturflaschen in der Inkubatorkammer des erfindungsgemäßen Inkubators wuchsen. Es wurden DH5a in CHO-CD-Medium gemessen und die aufgenommenen Messsignale der Gassensoren über die Zeit aufgetragen.

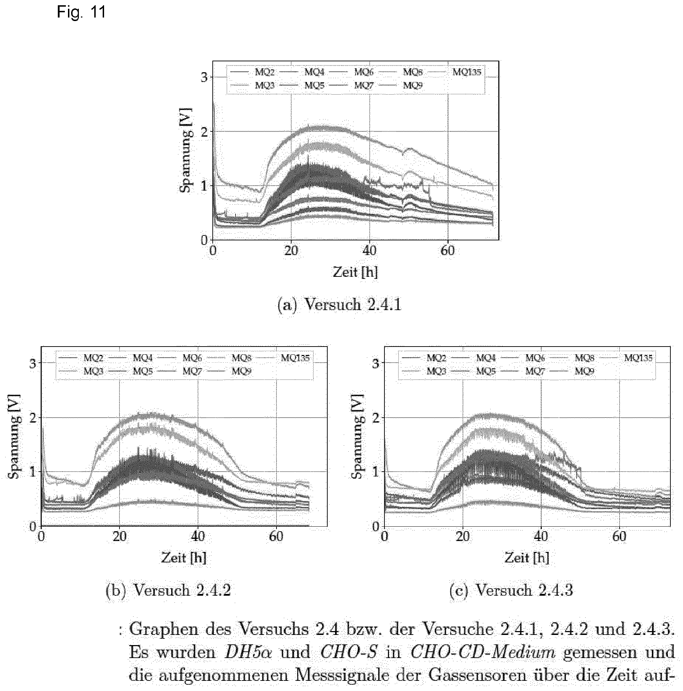

Fig. 11 zeigt Diagramme mit Messwerten zu einer Messung von VOCs aus DH5a und CHO-S in CHO-CD-Medium, die in Zellkulturflaschen in der Inkubatorkammer des erfindungsgemäßen Inkubators wuchsen. Es wurden DH5a in und CHO-S in CHO-CD-Medium gemessen und die aufgenommenen Messsignale der Gassensoren über die Zeit aufgetragen.

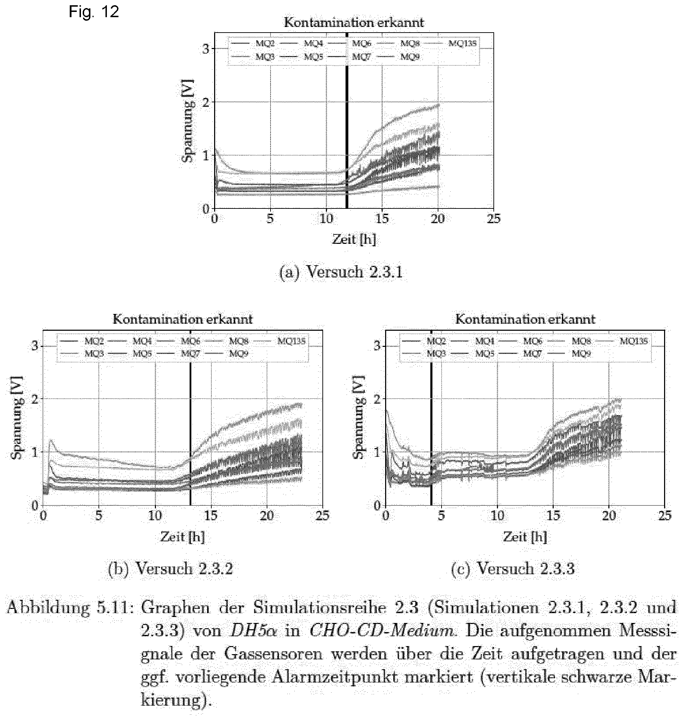

Fig. 12 zeigt Diagramme mit Messwerten zu einer Messung von VOCs aus DH5a in CHO-CD-Medium, die in Zellkulturflaschen in der Inkubatorkammer des erfindungsgemäßen Inkubators wuchsen.. Die aufgenommen Messsignale der Gassensoren werden über die Zeit aufgetragen und der ggf. vorliegende Alarmzeitpunkt markiert (vertikale schwarze Markierung).

Anhand der Versuchsergebnisse ist gezeigt, dass Sensoreinrichtung 61 in der Lage ist, eine anwachsende mikrobielle Kontamination einer Zellkultur zu erkennen. Das gassensorische System kann also dazu beitragen, dass mikrobielle Kontaminanten nicht unerkannt bleiben und somit weiterführende Probleme in den Anwendungsbereichen der Zellkultivierung vermieden werden. Die Integration des gassensorischen Systems in den CO2-Inkubator war erfolgreich und die Verwendung des gassensorischen Systems im Laborumfeld des CO2-Inkubators wurde erleichtert.

## Patentansprüche

1. Inkubator (1) zur Inkubation lebender Zellkulturen, aufweisend
eine Inkubatorkammer (2) zur Aufnahme von Objekten, insbesondere Zellkulturbehältern, in einem verschließbaren Innenraum der Inkubatorkammer, der eine kontrollierbare Gasatmosphäre aufweist,
eine Sensoreinrichtung (11; 21; 31; 41) zur Detektion einer Anreicherung, insbesondere Kontamination, von flüchtigen organischen Verbindungen (VOCs) in der Gasatmosphäre des Innenraums, wobei die Sensoreinrichtung mindestens einen VOC-Sensor (11) zur Detektion der VOCs aufweist und der mindestens eine VOC-Sensor mindestens einen Messbereich aufweist, der in Strömungsverbindung mit dem Atmosphärengas des Innenraums stehend angeordnet ist.

2. Inkubator gemäß Anspruch 1, der einen Strömungskanal, insbesondere mindestens eine Gasleitung aufweist, der aus dem Innenraum der Inkubatorkammer in eine in einem Außenraum der Inkubatorkammer angeordnete Messkammer führt, so dass Atmosphärengas aus der Inkubatorkammer in die Messkammer transportierbar ist.

3. Inkubator gemäß Anspruch 2, der einen Strömungskanal, insbesondere eine Abgasleitung aufweist, der angeordnet ist, um eine Abluft aus der Messkammer in einen Außenraum der Messkammer zu befördern.

4. Inkubator gemäß Anspruch 2, der einen Strömungskanal, insbesondere eine Rückführgasleitung aufweist, der angeordnet ist, um die Abluft aus der Messkammer, vorzugsweise durch einen Filter, insbesondere einen Hepa-Filter, zurück in die Inkubatorkammer zu befördern.

5. Inkubator gemäß Anspruch 2, wobei die Sensoreinrichtung eine Mehrzahl von VOC-Sensoren aufweist, deren Messbereiche, insbesondere deren Adsorptionsflächen, in Kontakt mit einem Innenraum der Messkammer angeordnet sind.

6. Inkubator gemäß Anspruch 5, wobei die VOC-Sensoren MOX-Sensoren sind und eine Heizseite aufweisen, die insbesondere jeweils außerhalb der Messkammer angeordnet ist.

7. Inkubator gemäß Anspruch 2, wobei die Messkammer einen torusförmigen Innenraum eines torusförmigen Messkammerabschnitts der Messkammer aufweist, wobei die Messbereiche mehrerer VOC-Sensoren entlang einer Wand des torusförmigen Messkammerabschnitts angeordnet sind.

8. Inkubator gemäß Anspruch 2, wobei die Sensoreinrichtung als elektronische Nase ausgestaltet ist und eine elektronische Steuereinrichtung sowie eine Mehrzahl von unterschiedlichen VOC-Sensoren aufweist, sowie insbesondere eine Spüleinrichtung aufweist, mittels der die Messkammer durch ein Spülgas gespült werden kann.

9. Inkubator gemäß Anspruch 8, wobei die Steuereinrichtung eine Datenverarbeitungseinrichtung mit mindestens einem Datenspeicher aufweist, die dazu programmiert ist, folgende Schritte auszuführen:
i) Speichern eines Messdatensatzes im einem Datenspeicher, der die, insbesondere zeitabhängig erfassten, Messwerte der Anzahl N>1 von VOC-Sensoren beinhaltet, wobei ein Messwert für das detektierte Messignal des jeweiligen VOC-Sensors charakteristisch ist, das in Gegenwart eines aus der Inkubatorkammer stammenden und am Messbereich des VOC-Sensors anliegenden Volumens der Gasatmosphäre erfasst wurde;
ii) Ermitteln von ersten Ergebnismessdaten aus einem Vergleich des Messdatensatzes mit einem Referenzdatensatz, insbesondere unter Verwendung einer Differenz des Messdatensatzes und des Referenzdatensatzes, der, insbesondere zeitabhängig erfasste, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung stammenden und am Messbereich des VOC-Sensors anliegenden Spülgases erfasst wurde;
optional: iii) Erkennen eines charakteristischen Datenmusters in dem die Ergebnismessdaten enthaltenden Ergebnismessdatensatz, wobei das charakteristische Datenmuster ein bestimmtes, im Atmosphärengas nachgewiesenes VOC, insbesondere auch dessen Konzentration oder Menge, repräsentiert.

10. Inkubator gemäß Anspruch 9, wobei der Schritt iii) beinhaltet, dass ein mittels maschinellem Lernen ermittelter Klassifikationsalgorithmus, insbesondere ein neuronales Netzwerk, zum Klassifizieren des charakteristischen Datenmusters verwendet wird.

11. Inkubator gemäß Anspruch 8, wobei die Steuereinrichtung eine Datenverarbeitungseinrichtung mit mindestens einem Datenspeicher aufweist, die dazu programmiert ist, folgende Schritte auszuführen:
i) Speichern eines Testmessdatensatzes in einem Datenspeicher, der, insbesondere zeitabhängig erfasste, Testmesswerte der Anzahl N>1 von VOC-Sensoren beinhaltet, wobei ein Testmesswert für das detektierte Messignal des jeweiligen VOC-Sensors charakteristisch ist, das in Gegenwart eines der Messkammer zugeführten und am Messbereich des VOC-Sensors anliegenden Volumens eines vorbekannten Testgases mit, insbesondere nach Art und/oder Menge, vorbekanntem VOC-Gehalt erfasst wurde;
ii) Ermitteln von zweiten Ergebnismessdaten aus einem Vergleich des Testmessdatensatzes mit einem Referenzdatensatz, insbesondere unter Verwendung einer Differenz des Testmessdatensatzes und des Referenzdatensatzes, der die, insbesondere zeitabhängig erfassten, Referenzmesswerte der Anzahl N>1 von VOC-Sensoren beinhaltet, wobei ein Referenzmesswert für das detektierte Messignal des jeweiligen VOC-Sensors charakteristisch ist, das in Gegenwart eines aus einer Spüleinrichtung entnommenen und am Messbereich des VOC-Sensors anliegenden Spülgases erfasst wurde;
iii) Speichern eines die zweiten Ergebnismessdaten enthaltenden zweiten Ergebnismessdatensatzes, der ein nun bekanntes, für das Testgas charakteristisches Datenmuster aufweist.

12. Inkubator gemäß Anspruch 11, wobei ein Schritt iv) vorgesehen ist, der beinhaltet, dass die zweiten Ergebnismessdaten als gelabelte Daten verwendet werden, um einen lernfähigen Klassifikationsalgorithmus durch maschinelles Lernen zu trainieren, insbesondere ein neuronales Netzwerk, der/das nachfolgend zum Klassifizieren gemessener charakteristischer Datenmusters verwendbar ist.

13. Inkubator gemäß einem der vorangehenden Ansprüche, der ein Informationsausgabesystem aufweist, insbesondere ein Display, einen Lautsprecher oder eine Datenschnittstelle zu einem externen datenverarbeitenden Gerät, um in Abhängigkeit von der mittels der Sensoreinrichtung erfassten Detektion von VOCs eine Information über diese Detektion auszugeben, insbesondere um eine Warninformation an einen Benutzer oder ein überwachendes System auszugeben.

14. Laborüberwachungssystem zur Erfassung der Anreicherung von VOCs, insbesondere zur Erfassung der Kontamination, in einer Inkubatorkammer, aufweisend
* mindestens einen Inkubator gemäß einem der vorangehenden Ansprüche;
* mindestens ein extern zu dem mindestens einen Inkubator angeordnetes, datenverarbeitendes Gerät, das insbesondere in einer Datenaustauschverbindung mit dem mindestens einen Inkubator steht, insbesondere über ein Intranet oder das Internet;
wobei das datenverarbeitende Gerät programmiert ist, die von dem mindestens einen Inkubator erhaltenen, mittels der Sensoreinrichtung des Inkubators durch die Detektion ermittelte Messdaten über eine mögliche Kontamination der Inkubatorkammer zu erfassen und in einer Datenspeichereinrichtung zu speichern, insbesondere um diese Messdaten an ein weiteres Gerät zu kommunizieren, insbesondere an ein Mobilfunkgerät.

15. Verfahren zur Erfassung der Anreicherung von VOCs, insbesondere zur Erfassung der Kontamination, in dem Innenraum einer Inkubatorkammer eines Inkubators, insbesondere eines Inkubators gemäß einem der Ansprüche 1 bis 14, aufweisend die Schritte:
* Erfassung von Messdaten mittels einer Sensoreinrichtung des Inkubators, die mindestens einen VOC-Sensor (11) zur Detektion von flüchtigen organischen Verbindungen (VOCs) aufweist, wobei der VOC-Sensor einen Messbereich aufweist, der in Strömungsverbindung mit dem Atmosphärengas des Innenraums stehend angeordnet ist;
* Feststellen einer möglichen Kontamination der Gasatmosphäre des Innenraums durch Auswertung der Messdaten.

16. Nachrüstbare Sensoreinrichtung zur Detektion einer möglichen Anreicherung von VOCs, insbesondere einer Kontamination, in der Gasatmosphäre des Innenraums einer Inkubatorkammer, wobei die Sensoreinrichtung mindestens einen VOC-Sensor (11) zur Detektion von flüchtigen organischen Verbindungen (VOCs) aufweist, wobei der VOC-Sensor einen Messbereich aufweist, der in Strömungsverbindung mit dem Atmosphärengas des Innenraums stehend anordenbar ist, und wobei die Sensoreinrichtung vorzugsweise eine Gasleitung aufweist, die zwischen dem Innenraum und dem Messbereich anordenbar ist, und vorzugsweise eine Pumpe, um ein Volumen der Gasatmosphäre des Innenraums der Inkubatorkammer durch die Gasleitung zum Messbereich zu befördern.

17. Inkubatoranordnung, aufweisend einen Inkubator mit einer Inkubatorkammer und einer Sensoreinrichtung gemäß Anspruch 16, die zur Detektion einer möglichen Anreicherung von VOCs, insbesondere einer Kontamination, in der Gasatmosphäre des Innenraums einer Inkubatorkammer, an dem Inkubator oder in der Inkubatorkammer des Inkubators angeordnet ist.
